# EUROPEAN PATENT SPECIFICATION

(11) **EP 2 142 181 B1**
(45) Date of publication and mention of the grant of the patent: **20.12.2017**
(21) Application number: 08735541.8
(22) Date of filing: 27.03.2008
(51) Int. Cl.: A61K 9/70, A61L 15/44, A61K 33/00

(54) **TOPICAL DERMAL DELIVERY DEVICE FOR NITRIC OXIDE DELIVERY**
TOPISCHE DERMALE ABGABEVORRICHTUNG ZUR ABGABE VON STICKOXID
DISPOSITIF DE DISTRIBUTION DERMIQUE TOPIQUE DESTINÉ À LA DISTRIBUTION D'OXYDE NITRIQUE

(30) Priority: 27.03.2007 WO PCT/EP2007/052923
(43) Date of publication of application: 13.01.2010
(73) Proprietor: BSN medical GmbH, 20253 Hamburg (DE)
(72) Inventor: LINDGREN, Lars, S-252 40 Helsingborg (SE); MÅRLIND, Linda, S-252 34 Helsingborg (SE); THORSEN, Rikard, S-224 74 Lund (SE)
(74) Representative: Jostarndt Patentanwalts-AG
(86) International application number: PCT/EP2008/053692
(87) International publication number: WO 2008/116925

(56) References cited:
- WO-A-02/20026
- WO-A-2006/073520
- WO-A-2006/084909
- WO-A-2006/084910
- WO-A-2006/100154
- WO-A-2006/100155
- US-A- 5 648 101

## Description

### Field of the Invention

This invention pertains in general to the field of non-implantable, topical delivery devices for topical delivery of a substance, compound or agent to a subject. More particularly, the invention relates to topical dermal delivery devices, such as patches, for topical delivery of nitric oxide (NO) to sites of mammals, for instance for therapeutic or cosmetic treatment at said site.

### Background of the Invention

Various compounds and delivery devices for topical treatment of numerous conditions are known. In WO 2006/084909, WO 2006/100155, WO 2006/084910, or WO 2006/100154 of the same applicant as the present application, related to topical nitric oxide delivery systems, and to using the same for mitigating or remediating various disease states. A device having a membrane system involved in NO delivery is disclosed. However, the efficiency of the disclosed device may be further improved, for instance with regard to the efficiency of the topical delivery system.

In addition, if such a topical delivery device is desired to be stored, extreme measures are needed to make sure that NO is not released premature during storage, e.g. by ensuring a non-moist, non-oxygen and dark storage. Many materials usually contain enough moisture themselves to initiate a premature NO release from an NO donor. Thus, shelf life of such a device may be limited.

A further issue is that is desired to have improved patches allowing delivery of a defined amount of NO, but with a more effective transfer at the target site. This is for instance of interest to counteract the very short half life of NO.

Also, a more efficient surface release in every portion of the patch is desired.

A patch is disclosed in WO2006/073520, for insuring long shelf life of unstable drugs, and comprises a compartment to retain an active mixture of drugs. Upon delivery of the active drugs a fluid may be injected to the reservoir to gain a solution capable of penetrating a layer towards the skin. However the device disclosed is not suitable for delivery of NO, as NO is an active substance and has a very short time span until it reacts, i.e. NO has a time limit in its active therapeutic phase. Hence, long term storage of the active drug as disclosed in WO2006/073520 until the active drug will be released is not possible. Furthermore, the disclosed device appears not to be suited for delivery of a gaseous active compound.

In US5648101, by R. Tawashi, several types of NO-releasing delivery systems are disclosed. NO is released in a chemical reaction process between a soluble salt and a nitrite. The amount soluble needed to activate the chemical process is according to US5648101 in the range of 50 to 100 microlitres. The amount makes it cumbersome for the general population to administrate. US5648101 does not disclose anything about contact size of the skin target area to be treated.

WO 2006/100155 relates to a device for wound care and teaches the use of a nitric oxide eluting polymer integrated in a carrier material and activated by a proton donor to release the NO. According to the WO 2006/100155 the NO-eluting polymers and the proton donor is provided in two different compartments such as e.g. in two separate containers of a syringe or with a proton donor-bag to be pushed in order to release the proton donor or with a micro-encapsulated proton donor whereby a compressing or squeezing results in breakage of the micro capsules so that the NO eluting polymer is exposed to the proton donor.

In sum, application of known compounds generating NO for therapeutical treatments has been impractically complicated and time consuming up to now. In addition, many of the NO generating systems are very sensitive, e.g. to humidity, oxygen, and light, and have to be stored under special conditions. This makes it very difficult to integrate the NO donors in products that have a shelf life allowing advantageous industrial applicability.

Moreover, known devices show a number of disadvantages with regard to topical or transdermal delivery of NO from an NO donor. In practice it may be difficult to administer NO topically to a subject, as e.g. mechanical influences e.g. by movements of the subject lead to an insufficient or ineffective delivery of NO. Also, activation, e.g. by pressure on the device may be difficult to achieve due to the softness of the skin.

Hence, an improved system or device for topical application of NO would be advantageous, and in particular a topical dermal delivery system allowing for instance one or more of increased flexibility, cost-effectiveness, efficiency of nitric oxide storage and/or transfer to a treatment site, storage time, convenience of use, patient friendliness, efficiency of therapy, and/or patient safety would be advantageous.

### Summary of the Invention

Accordingly, embodiments of the present invention preferably seek to mitigate, alleviate or eliminate one or more deficiencies, disadvantages or issues in the art, such as the above-identified, singly or in any combination by providing a topical dermal nitric oxide delivery device, a method of production, and a method of cosmetical treatment that allows for advantageous treatment, according to the appended patent claims.

According to some embodiments of the invention, a topical device that directs the elution of NO towards a treatment site is provided. According to some embodiments of the invention, an absorbing material in the device contains an NO donor compound and ensures that minimal leakage of unwanted components or undesired species from the device occurs.

According to a first aspect of the invention, a topical nitric oxide delivery device is provided as defined in claim 1.

According to another aspect of the invention, a use of nitric oxide in the manufacture of a medicament is provided.

According to another aspect of the invention a method of topically delivering nitric oxide is provided. The method comprises activating nitric oxide release from a nitric oxide donor system in a nitric oxide delivery device according to the first aspect of the invention; placing the activated nitric oxide delivery device in contact with a skin area of a body, with a nitric oxide permeable layer of the delivery device oriented towards said skin area; and delivering nitric oxide topically from said nitric oxide delivery device to said skin area via said nitric oxide permeable layer, wherein a permeability of said nitric oxide permeable layer is selectively chosen depending on a toxicity level of said nitric oxide donor system.

According to the invention, the topical nitric oxide delivery device can be used to deliver nitric oxide to a therapeutic treatment site, such as a site of peripheral neuropathy, diabetic ulcers, wounds, skin infection, fungal dermal attack such as onychomycosis, mixed dermal infections, and/or virus attack such as warts.

According to a further aspect of the invention, a cosmetical method is provided. The cosmetical method comprises delivering nitric oxide according to the above aspect of the invention to a cosmetic treatment site, such as a site of scars to be reduced by said nitric oxide, or a site of sagged skin or wrinkles to be lifted by said nitric oxide.

Features for the second and subsequent aspects of the invention are as for the first aspect mutatis mutandis.

Further embodiments of the invention are defined in the dependent claims.

Some embodiments of the invention provide for patient friendly, convenient delivery of nitric oxide to a skin area.

In some embodiments, a gap between a topical delivery device and an adjacent skin area to be treated may be eliminated, thus improving the efficiency of NO transport from the device to the skin. In some embodiments this is provided by improving the topical delivery device, such as a patch, with a specific layer configuration. Improved topical delivery devices having a more efficient treatment area, than conventionally, are thus provided. Thus it is provided to efficiently maintain an NO dosage delivered, or to improve the amount of deliverable active NO per surface area.

Some embodiments may comprise a smaller treatment area with a similar efficiency than comparatively larger conventional topical delivery devices.

In embodiments, the topical dermal delivery devices are provided for non-systemic local treatment of ailments.

In embodiments, the topical dermal delivery devices are provided without assistance of an active delivery system.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

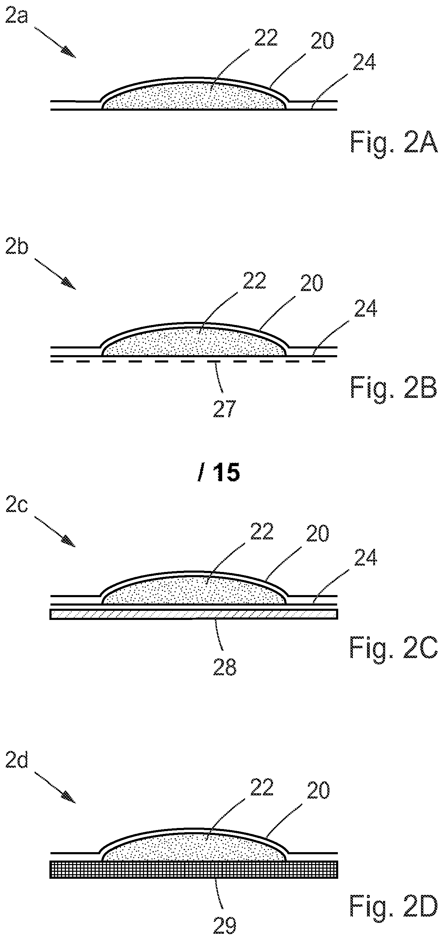

These and other aspects, features and advantages of which embodiments of the invention are capable of will be apparent and elucidated from the following description of embodiments of the present invention, reference being made to the accompanying drawings, whereby the specific embodiments disclosed in Figs. 2-11 and 13-16 do not fall under the granted claims, in which
Fig. 1 is a schematic illustration of a topical patch that has a multi layered structure,
Figs. 2A to 2D are cross sectional views of embodiments of patches having various NO permeable layers;
Figs. 3A to 3C are cross sectional views of embodiments of further patches having various arrangements of an NO impermeable backing layer;
Fig. 4A is a perspective view of a patch having a sealingly closable backing enabling fluid access to the patch interior during activation;
Fig. 4B is a cross sectional view of the patch shown in Fig. 4A;
Fig. 5 is a is a schematic illustration of a topical 10 patch that has a multi layered structure;
Fig. 6A is a perspective view of a patch having an access port to the interior of the patch system;
Fig. 6B is a cross sectional view of the patch shown in Fig. 6A;
Fig. 7A is a perspective view of a patch having a sealingly reclosable backing enabling exchange of a patch system component during use;
Fig. 7B is a cross sectional view of the patch shown in Fig. 7A as well as a further patch system component comprising an exchange component of the patch system;
Fig. 8 is a schematic illustration of a patch system component comprising an NO donor component and an activation fluid in a breakable receptacle;
Fig. 9 is a schematic illustration of a patch system component comprising an NO donor component and an activation agent in a multiple compartment arrangement;
Fig. 10 is a schematic illustration of a patch system component comprising an NO donor component and an activation agent in a multiple compartment arrangement of a bottle;
Fig. 11A is a perspective view of a patch system comprising a patch and the patch system component of Fig. 9 prior to admixing the NO donor component and a release activation fluid;
35 Fig. 11B is a perspective view of the patch system of Fig. 11A after admixing the NO donor component and a release activation fluid and filling the patch with the activated mixture;
Fig. 12A is a perspective view of a further patch system comprising an NO donor component and an activation agent in a multiple compartment arrangement;
Fig. 12B is a cross sectional view of the patch system shown in Fig. 12A along a line A-A;
Fig. 13 is a perspective view of a further patch system comprising an NO donor component and an activation agent in a breakable multiple compartment arrangement;
Fig. 14 is a perspective view of a patch having multiple compartments;
Fig. 15 is a perspective view of a patch having passageways for a fluid to and from the skin to which the patch is applied in use;
Fig. 16A is an exploded view of an embodiment of a patch and Fig. 16B is a perspective view of the patch;
Figs. 17A and 17B are flow charts illustrating embodiments of a manufacturing procedure for a patch;
Fig. 18 is a graph illustrating a substantially maintained fluid retention of a patch with varying pressure applied; and
Fig. 19 is a graph illustrating the NO transfer efficiency of different patches.

### Description of embodiments

Specific embodiments of the invention will now be described with reference to the accompanying drawings. This invention may, however, be embodied in many different forms
and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to a topical patch, and in particular to a topical patch releasing NO. However, it will be appreciated that the invention is not limited to this application but may be applied to many other delivery devices, including for example wraps, bandages, etc.

The base of the topical NO delivery system is the absorbent core or reservoir in which the active mixture of NO donor and activation fluid is provided upon activation of the delivery system for use. This to provide an even spread of the donor over the device, to give a stable containment of the donor and to assure an even release during the treatment.

NO donor systems that comprise both a NO donor as well as an activation solution may in some cases be irritating to the skin and therefore a selective membrane can be placed in between the absorbent core and the skin. Skin irritation is disadvantageous for the patient and should be overcome. This is for instance done by avoiding skin contacts of irritating, undesired species.

A mechanical property of the NO delivery system components of some embodiments, such as the NO permeable membrane, is that they are flexible and therefore are able to adapt to the skin surface to assure good contact.

The NO releasing membrane material is selectively permeable for NO that is allowed to penetrate to the skin while passage of unwanted components or undesired species via the membrane is minimized.

In order to direct the NO release towards the treatment site, a top cover of a non-permeable material.

This cover also increases the concentration of NO in the fluid since the partial pressure of NO in gas state is increased.

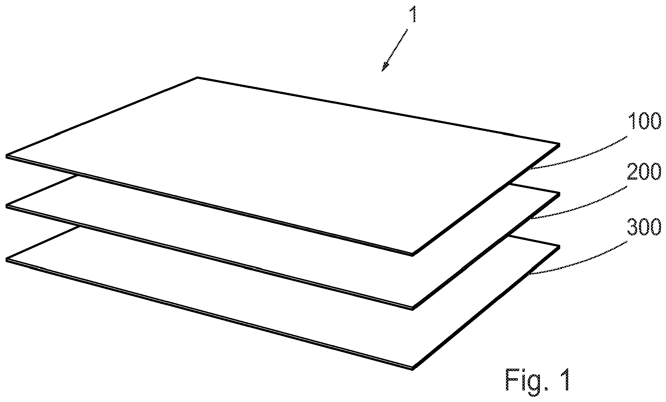

As illustrated in Fig. 1, embodiments of the topical patch 1 have a layered structure comprising an NO permeable layer 300, such as a membrane, in use of the patch oriented towards the area to be treated, and an NO non-permeable layer 100, i.e. a layer that is substantially not permeable for NO, such as a suitable impermeable membrane or film, as a backing. Between these two layers, an absorbing or reservoir layer 200 is provided, also called an absorbing core. The absorbing core may be provided in the form of a fluid retention system in order to minimize leakage and to provide a reservoir for an NO donor.

Further below, a number of embodiments will be given with regard to each of the NO permeable layer 300, the absorbing layer 200, and the non NO permeable layer 100. Furthermore, additional layers being arranged adjacent to these layers will be described in connection with some embodiments.

When activating release or elution of NO from the NO donor, or more specifically from an NO donating compound, provided at the absorbing core, the released NO diffuses within the patch 1. Some embodiments of suitable NO compounds are given further below. NO may be provided in gaseous form or dissolved in a liquid. Since the direction of the diffusion is controlled by the principle of minimum diffusion resistance, i.e. diffusion takes place in the direction of the least hinder, NO only escapes substantially through the NO permeable layer 300. The NO permeable layer 300 is for instance a membrane selectively permeable for gaseous NO or dissolved NO. Thus, in use of the patch, the NO released inside the patch, is directed towards the skin of the patch user through the NO permeable membrane. The NO release is effectively targeted towards the treatment site.

### NO non-permeable layer 100

In addition, the non NO permeable layer 100, during use oriented away from the patient, i.e. on the outer side of the patch, does not only contribute to directing the released NO towards the skin, it also contributes to increasing the partial pressure of NO inside the patch and therefore increases the concentration of dissolved NO therein. The NO non-permeable layer 100, such as a membrane non-permeable for NO, serving as a backing layer, is arranged and configured to limit the loss of concentration during the production of NO in the patch as well as see to that the NO concentration or partial pressure increases, at least during the initial phase of NO release. In such a manner, the backing layer may for instance assure an increased amount of dissolved NO within the patch.

The non NO permeable layer 100 may in use also prevent that a liquid leaks out of the patch, which may comprise an effective prevention of body fluid leakage through the non NO permeable layer 100, including blood or exudate from wounds.

Backing layer 100 may be made of a barrier material or layer, such as a metallized polymeric film, or a metallic film, such as an aluminium film.

In addition backing layer 100 may comprise inlet ports, such as Luer lock ports for access to the interior of the patch.

### Absorbing layer 200

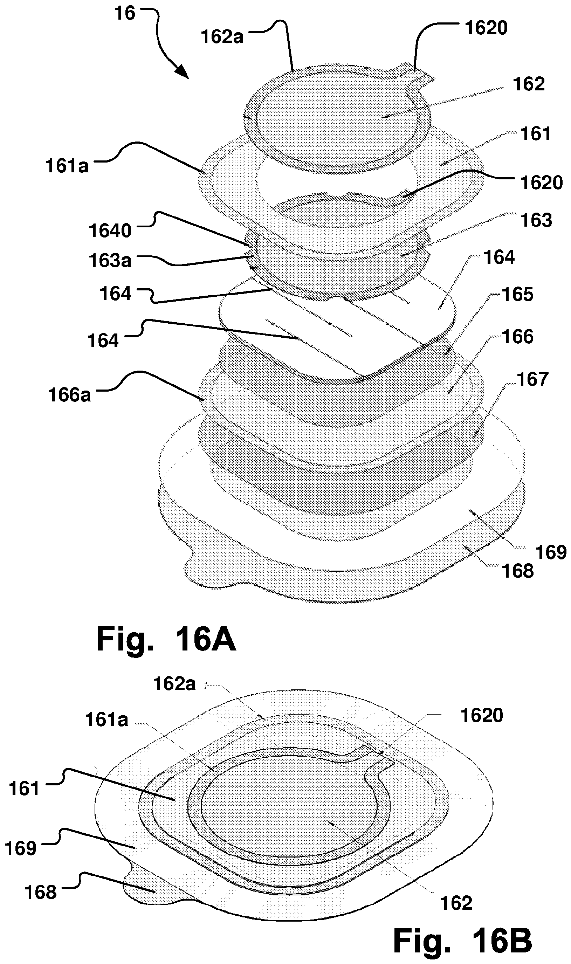

The absorbing layer 200, i.e. the absorbing core, may have multiple functions during the use of the patch, e.g. may be integrated with or comprise an NO donor such as described with respect to Fig. 16, which however shows an embodiment that is not part of the invention. The NO donor may be comprised in the absorbing layer in an inactive stage for activation with a fluid.

The layer 200 is arranged to store and retain a nitric oxide releasing fluid in use of the patch. Furthermore, it also interacts with the NO donor compound to ensure minimal migration of the donor out from the patch. In the case of precipitation of the NO donor after NO release, the absorbent efficiently traps and contains such possible residues. Another positive property of the absorbent system is that it ensures that the NO donor has continuous contact with the activation system and thus ensures a continuous NO release. A fluid comprises a nonsolid fluid, including at least partly a gel or a liquid portion, which have in common that they are substantially incompressible, which contributes to the advantageous effects of some embodiments, such as a loss of said interfacial area between a nitric oxide permeable layer and a treatment area is substantially avoided in use of the topical delivery device.

In an embodiment, the absorbent core is provided in form of an absorbing system, e.g. a gel based system generated by a super absorbent polymer, such as super absorbent powder (SAP) and/or super absorbent fibers (SAF) or equal gelling material, that absorbs and retains the fluid. The absorbing component may be incorporated with other materials known to the skilled person, e.g. for providing wicking, mechanical strength, etc. In order to enhance shelf life of the patch, the NO donor compound is not activated for NO release prior to use. That means, even if the NO donor compound is arranged in a non-activated form, the above described gel based system is activated during storage. It is not feasible to maintain an NO donor compound in a gel form for long-term storage, prior to use, in a patch. When providing drugs, according to the prior art, in gels as well as different reservoirs, this is done for permanent storage of the drug in the reservoir focusing on containing it in a stable form. This stands in contrast to the required targeted activation of NO release upon use of the patch. The reservoir in the present application has a function during the actual NO release and in that case being a storage and entrapment for both the activation fluid as well as the donor in a reservoir that contains these substances in a fluid stage. The fluid stage may be a gel structure generated by a super absorbent polymer SAP/SAF or equal gelling material that contains the fluid within its boundaries.

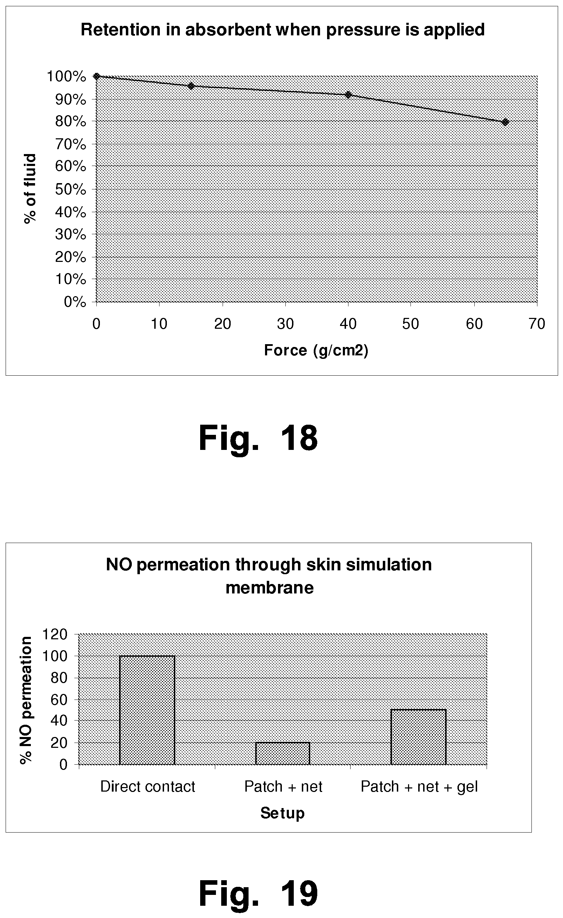

The retention in the absorbing core ensures stable NO release even at physical deformation, such as bending, applying uneven pressure, tilting etc. When a point pressure is applied onto a fluid containing reservoir the fluid wants to move away from where the pressure is applied, the retention in the absorbent acts as a opposite force and keeps at least some fluid in the area. When pressure is applied onto a fluid filled reservoir without an absorbing core, 0% of the fluid is kept. When pressure is applied onto a fluid filled absorbent core most of the fluid is kept when the pressure is applied. How much is kept depends on the pressure. In an example, illustrated in Fig. 18, when 0-40 g/cm2 pressure is applied on to an absorbent more than 90% of the fluid is kept and for pressure 40-65 g/cm2 more than 80% of the fluid is kept.

The above feature ensures the distribution of the fluid in the absorbent and thus gives a high interfacial area between the fluid and nitric oxide permeable layer even when exposing the device to environmental stress such as bending, applying uneven pressure, tilting etc.

Advantageous NO transport from the patch is provided when the absorbent core is in good contact, i.e. has a high interfacial area, with the membrane that is NO permeable towards the area to be treated. In this way good NO transport to the treatment site is ensured. One way to obtain this is to have the absorbent close to full wettening so that the fluid trapped in the system comes in good contact with the membrane. Full wettening is achieved in dependence on the absorbent system and/or the NO donor system, and may be achieved at rates exceeding 60% fluid content thereof, e.g. more than 80% or more than 90%. These percentages refer to that measured of free swell. Another way is to glue the membrane on the absorbent.

The above-mentioned NO reservoir system based on an absorbent core, assures that NO release from the patch is maintained throughout the whole treatment and that both the activation fluid and the NO donor are safely contained within the patch.

Thanks to the advantageous NO production in the patch and the use of a gel, it is assured that NO that is generated in a liquid stage is maintained in that stage before diffusing towards the treatment site. Furthermore, the NO donor compound itself, which may be toxic, is contained and retained in the patch, thus avoiding that the NO donor comes into contact with the skin. In addition, when the NO donor in specific cases precipitates in the patch after or during NO release, any residues of the precipitation are trapped in the patch, and can thus not come into contact with the skin.

Applicants have found that the efficiency of a topical patch is increased with dissolved NO in comparison to gaseous NO. The backing contributes to increasing the partial pressure of NO inside the patch and therefore increases the concentration of dissolved NO therein which is more efficient than gaseous NO. The distribution of NO donor prior to activation also increase the possibility of high NO concentration due to minimizing local over saturation and thus minimizing NO gas formation. Furthermore, NO release from the patch is most effective at a fluid content relationship of the absorbent core at more than 60% fluid content thereof, e.g. more than 80% or more than 90%, depending on the fluid contact with the NO permeable membrane.

Pure gas treatments, e.g. as described in US2004/0009238, require very high concentrations of NO in the delivery gas, e.g. 200 ppm or more. Such high concentrations could be dangerous for the user, if for instance inhaled by mistake.

Other suitable reservoir systems might be provided with some embodiments, i.e. not only an absorbent core of a matrix material. Alternatively, also a fluid, viscous fluid or gel may be provided in some embodiments.

### NO donor systems

Some embodiments of NO donor systems for generating NO in the patch may be based on compounds comprising polymers comprising an 0-nitrosylated group, such as diazeniumdiolate groups, S-nitrosylated and 0-nitrosylated groups, or any combinations thereof.

The nitric oxide (NO) eluting polymer may comprises diazeniumdiolate groups, S-nitrosylated groups, and 0-nitrosylated groups, or any combination of these.

Some embodiments of NO donor systems for generating NO in the patch may be based on NONOates.

Some embodiment may be provided with a nitrite/acid system for delivery of NO.

Activation fluid includes proton donors and comprise without limitation liquids such as water, physiological buffers, and saline. The activation fluid activates release of nitric oxide from an nitric oxide donor, like the aforementioned compounds, in the NO delivery device 1 for topical delivery.

### NO permeable layer 300

The NO permeable layer 300, comprises a material compatible with NO, such as a membrane, in use of the patch oriented towards the area to be treated, is devised to regulate the diffusion of NO, e.g. dissolved or gaseous NO, out of the patch towards the skin. Thus, the NO permeable layer 300 is configured to regulate the NO permeation rate from the patch to the user's skin.

Furthermore, the membrane may be chosen in dependence on the NO generating system in the patch. For instance, the NO generating system may comprise NO donor compounds or degradation products that are toxic, irritant, or nontoxic. The NO permeable layer 300 may be chosen in dependence of this level of skin compatibility, i.e. different membranes may be suitable. In this manner, the patch may be provided in a cost-efficiently manner while maintaining patient comfort.

In the case of good communication, e.g. conventional filtration, allowed across the NO permeable layer 300, e.g. when the NO generating system is non-toxic, the NO permeable layer 300 may be made in form of a membrane of nonwoven or porous materials permitting fluid flow but retaining larger particles. In this case, the NO released from the NO generating system may be transported dissolved in the fluid across the membrane to the skin.

In the case of medium communication, being allowed across the NO permeable layer 300, e.g. when the NO generating system is irritant, and in case the activation fluid is water, a high moisture vapor transfer rate (MVTR) material, such as a micro porous membrane or e.g. a hydrophilic membrane composed of a partially hydrophilic block co-polymer membrane, may be provided in some embodiments. Such membranes may allow transport of activation fluid including the dissolved NO and other nontoxic nonirritant low molecular weight substances across the membrane but retain larger molecules e.g. the NO generating system.

In the case of low communication is allowed across the NO permeable layer 300, e.g. when the NO generating system is moderately toxic, it is necessary to hinder medium to large components from traveling across the NO permeable layer 300. In case the fluid is water, a medium/low MVTR material may be chosen to permit transport of NO and water while transport of toxic and irritant substances is prohibited.

In the case no communication is allowed across the NO permeable layer 300, e.g. when the NO generating system is toxic, it is necessary to totally separate the interior of the patch system from the skin with regard to the NO generating systems. The material of the NO permeable layer is chosen such that it is highly selective towards NO transport, e.g. operating via a size selectivity mechanism, allowing no liquid communication across the membrane. Any liquid transport is fully prevented by the membrane. In case the fluid is water, at least one layer is provided in the membrane, which is hydrophobic, in order to hinder water transport across the membrane. In this case, NO is transported up to the hydrophobic layer dissolved in the water. From the hydrophobic layer, withholding the fluid water, NO diffuses from the water through the hydrophobic layer towards the skin.

Suitable membrane NO compatible materials are Silicones, Co-polyester, polyolefins, polyimides, polysulfones, polamides, EVA, PTFE, as well as PUR that has sufficient permeability to be used, depending on configuration and material with regard to providing selectively fluid or water permeation or not. The membrane's permeability does not degrade with contact of NO or NO reaction products.

Hitherto membranes have been permeable for substances of a molecular weight higher than NO. NO permeable layer 300 is configured to be selectively permeable for nitric oxide molecules. In this manner the NO permeable layer 300 provides for an efficient protection barrier preventing unwanted NO donor compound components, to be transferred via the NO permeable layer 300 during the transfer of NO.

Hence, the NO permeable layer 300 not only regulates the NO release from the patch, is also ensures that the NO donor compound or components thereof, are only exposed to the skin if allowed, e.g. when non-toxic or non-irritant. US2005142218 of Benjamin et.al discloses a barrier consisting of a membrane that allows diffusion of nongaseous nitrate ions while preventing direct contact of the skin and an acidifying agent. The membrane disclosed in US2005142218 comprises a pharmacologically acceptable acidifying agent and a pharmacologically acceptable source of nitrite ions or a nitrite precursor therefore. The membrane may be fully-, or partially-permeable, including semi-permeable or selectively permeable, to the passage of nitric oxide ions. Such membranes can prevent direct contact of the composition with the skin but can permit diffusion of nitric oxides into the skin. This means that the membrane disclosed in US2005142218 is not selectively permeable for NO, and does not control that an NO donor compound or components thereof, are not exposed to the skin. Furthermore, directed, efficient application of NO to a skin site is not disclosed. Moreover, fluid access into the patch is not provided.

A further positive feature of embodiments of the NO permeable layer 300, such as membrane, is that transport of NO donor compounds through the membrane is minimized.

### Skin Contact Enhancer

The NO permeable layer 300, compatible with NO and non-degradable with NO, in use of the patch oriented towards the skin of the patch user, may comprise a skin contact enhancement layer or have arranged an adjacent skin contact enhancement layer, or it may have such contact enhancement properties that it provides sufficient skin contact properties by its intrinsic suitable properties.

The contact to the skin can be enhanced by an adhesive layer, such as a silicone gel layer, a pressure sensitive adhesive (PSA), or a hydrogel layer. The skin contact enhancement layer provides good contact between the membrane and the skin. In embodiments, the adhesive layer is continuous and is configured to fill out unevenness in the skin. Unevenness may be caused by various reasons, such as scar tissue, body hair etc. Furthermore, the skin contact enhancement layer provides enough adhesion capabilities to stick the patch to the skin. As unevenness in the skin is filled out, leakage of nitric oxide away from the patch instead of into the skin is prevented, thus rendering the patch even more advantageous and delivery of NO more effective.

As illustrated in Fig. 19, the applicants have found that if a patch with an NO permeable membrane with good contact properties: close to 100% interfacial contact, is applied onto a skin simulation membrane an efficient NO permeation is achieved. From here on this efficient permeation is referred to as 100% NO permeation. When such an identical patch is applied onto the same skin simulation membrane, but with a net between the patch and skin simulation membrane, in order to distance the patch and minimizing the contact with the skin simulation membrane, less than 20% of the original NO permeation is achieved. But when an identical patch is applied onto the skin simulation membrane with a net and with a gel filling out the distance between the skin simulation membrane and the patch, and thus creating improved contact properties, more than 50% of the original NO permeation is achieved.

The skin contact enhancement layer has to be permeable for NO and non-degradable with NO. Both a good skin contact of the patch and a sufficient transport of NO through the skin contact enhancement layer are provided by the patch according to the embodiments described herein. Previously, it has not been not taken into account that the NO transport through such a skin contact layer is an issue. The skin enhancement layer needs to be sufficiently permeable for NO in order to provide a patient friendly and effective release of NO from the patch to the skin of the user. This issue may be resolved by attaching the skin contact layer to the NO permeable membrane on the side that in use is turned towards the skin. Further below, suitable materials and other features for an optimal NO transport through an NO permeable layer 300 and/or a skin contact enhancement are described in more detail.

Some embodiments comprise a skin contact layer that may be used repeatedly for continuous therapy, wherein such embodiments may comprise an absorbent exchange system that is replaceable after a certain time of use with a fresh one without removing the patch from the skin of the user.

### Fluid direction system

Some embodiments of the patch may comprise a fluid direction system including an NO donor compound. The fluid direction system is arranged to receive an activation fluid and direct the activation fluid to an NO donor compound, whereupon the activation fluid dissolve the NO donor compound prior to applying it into the absorbent core. Some embodiments comprise a fluid direction system in form of a spread systems as well as connection points to the patch for receiving the activation fluid. For instance, a single injection point for introducing the activation fluid into a sealed patch is provided. In the patch, the fluid is absorbed as described. The fluid direction system may be integrated into the absorbent layer, such as shown in Fig. 16. In addition, the fluid direction system may be provided such that the structural characteristics of the embodiments are enhanced. For instance, longitudinal slits 164 may be provided for fluid direction, improving flexibility of the patch.

### Storage of the patch

In some embodiments, the challenge to ensure safe long term storage of the patch system, or at least of the NO donor compound thereof, is solved by individual storage of the different components. By storing the sensitive NO donor compound in a separate compartment within or separate from the device, it is ensured that the necessary parameters are fulfilled. The environmental parameters that may affect the NO donors are for example moisture, oxygen, and light. On the other hand, the separation of some individual components of the patch system puts high requirements on the design so that the integration of the components may be performed in simple, user-friendly, reliably and efficient manner. In some embodiments, this challenge is solved by means of a sealingly reclosable, or closable opening, such as an activation window, provided in the backing of the patch, i.e. in the NO non-permeable layer of the patch. Such an activation window enables an exchange of at least a part of the absorbent core, for instance when the NO release has decreased. This provides to sustain the NO release from the patch over a long period of time without changing the membrane towards the skin, which is an advantage for the user as the skin is less stressed by removing the patch less frequently from the skin.

In addition, fluid access into the device is provided according to some embodiments, e.g. by a fluid communication port. This allows a patch to be stored for a long term, e.g. under dry, oxygen free and/or dark conditions, whereupon activation of NO release from the patch is provided via introducing a suitable fluid into the patch via such a fluid communication port.

Some specific embodiments will now be described in more detail.

### Topical patch

Topical patch systems for therapeutic or cosmetic treatment with nitric oxide is provided in a number of embodiments, as illustrated in Figs. 2A-2D, 3A-3C, 4A and 4B, 6A and 6B, 7A and 7B, 11A and 11B which all show embodiments that do not fall under the scope of the granted claims. Details of these embodiments are illustrated by means of the remaining Figs.

Figs. 2A to 2D showing embodiments being not part of the invention are cross sectional views of embodiments of patches 2a-2d having a fixed backing layer 20 and an absorbent core or reservoir 22 from which in use NO is provided for treatment. Patches 2a-2d have different arrangements on the patient side of the patch. Patches 2a, 2b, and 2c comprise a NO permeable membrane layer 24. Patch 2d comprises an NO permeable gel 29. Patch 2b comprises a pressure sensitive adhesive (PSA) skin contact enhancement layer. Patch 2c comprises a silicone gel layer 28 serving as a skin contact enhancement layer. Patch 2d comprises a gel membrane 29.

The layers of patches 2a-2d are shown apart for illustrative purposes only. Assembled patches 2a-2d are provided with the layers in close contact, in such a manner that the function of efficient topical NO application is provided. Patches 2a-2d also comprise adhesive joints or layers, which are not illustrated, in order to suitably attach the layers to each other. For instance, backing layer 20 is glued to membrane 24 around the absorbent core or reservoir 22. Backing layer 20 may also be glued to the absorbent core or reservoir 22.

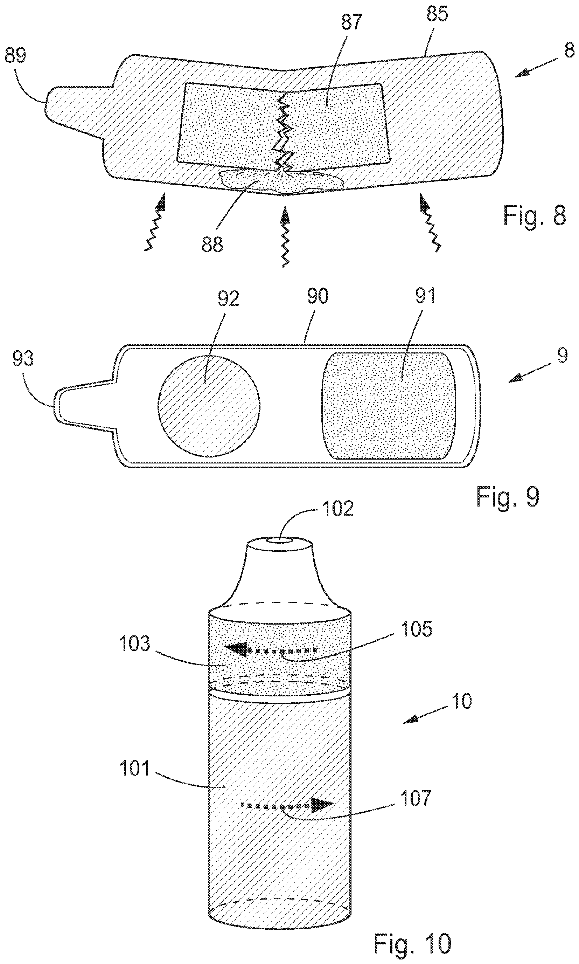

Patches 2a-2d comprise both an NO donor compound and an activation system suitably separated from each other during storage. Suitable ways of separation are described further below, for instance with reference to Figs. 8, 9 or 12, e.g. by having the NO donor in a glass ampoule that is broken upon use in order to create a contact of the NO donor and an activation fluid, e.g. a SAP/SAF absorbent core that is saturated with more than 90% water.

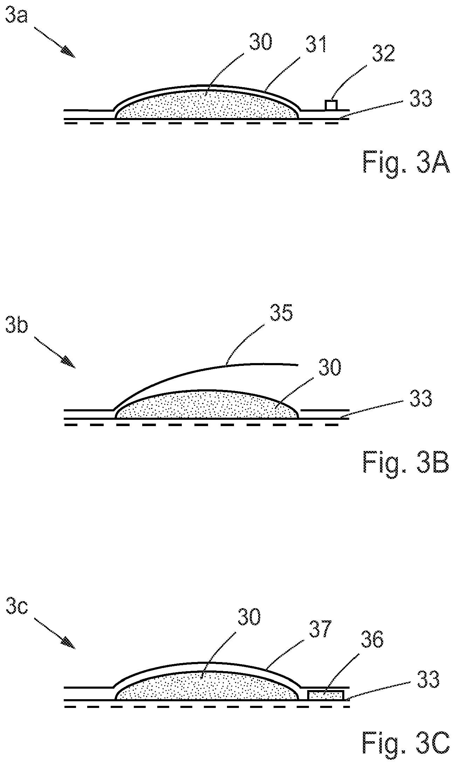

Figs. 3A to 3C showing embodiments being not part of the invention are cross sectional views of embodiments of further patches, wherein variations of the absorbent core or reservoir, or of the backing layer are illustrated. Patches 3a-3c are illustrated having an NO permeable membrane layer 33. As indicated by the dashed line in Figs. 3A-3D, patches 3a-3c may also comprise additional or alternative skin enhancement layers, or combined skin enhancement layers and membranes, such as the ones illustrated in Figs. 2A-2D, for instance a permeable gel membrane etc. As in Figs. 2A-2D, the layers of patches 3a-3c are shown apart for illustrative purposes only. The patches 3a and 3b have a backing layer 31, 35, respectively, which provide access to the absorbent core or reservoir 30 from which in use NO is provided for treatment. Patches 3a-3c may have an NO donor compound incorporated into the absorbent core or reservoir 30. As access is provided to the interior of the patch, an activation fluid may be introduced into the interior of the patch for activation of NO release from the NO donor compound. Patch 3a is provided with an access port 32 in an impermeable backing 31 to a pouch in the interior of patch 3a, comprising the absorbent core or reservoir 30. Through port 32 communication is given with the interior of patch 3a. Port 32 is equipped with suitable units ensuring that fluid only enters the patch and leakage is avoided, such as one way flap valves. Through port 32 an activation fluid may be introduced into patch 3a for activation of release of No from an NO donor contained therein, e.g. incorporated into an absorbent core. An activated, pre-prepared NO releasing fluid may also be introduced through port 32 into patch 3c for NO release therefrom. Patch 3b comprises a backing layer 35, which provides access to the absorbent core or reservoir 30. In this way, like through port 32, either an activation fluid or an activated NO releasing fluid may be introduced into the patch 3b. Backing layer 35 is sealingly closed after introducing the fluid into the patch 3b. A suitable adhesive, e.g. underneath a release liner that is removed prior to closing the lid, is provided on the inner surface of the flap of backing layer 35, such that an NO impermeable backing is provided after closing the lid. Patch 3c comprises a fixed backing, and a system of separated NO donor compound and activation fluid, which is arranged such that the NO donor compound and the activation fluid may be brought into contact by outer action, e.g. by removing an intermediate impermeable membrane. Further details for this type of patch are given below.

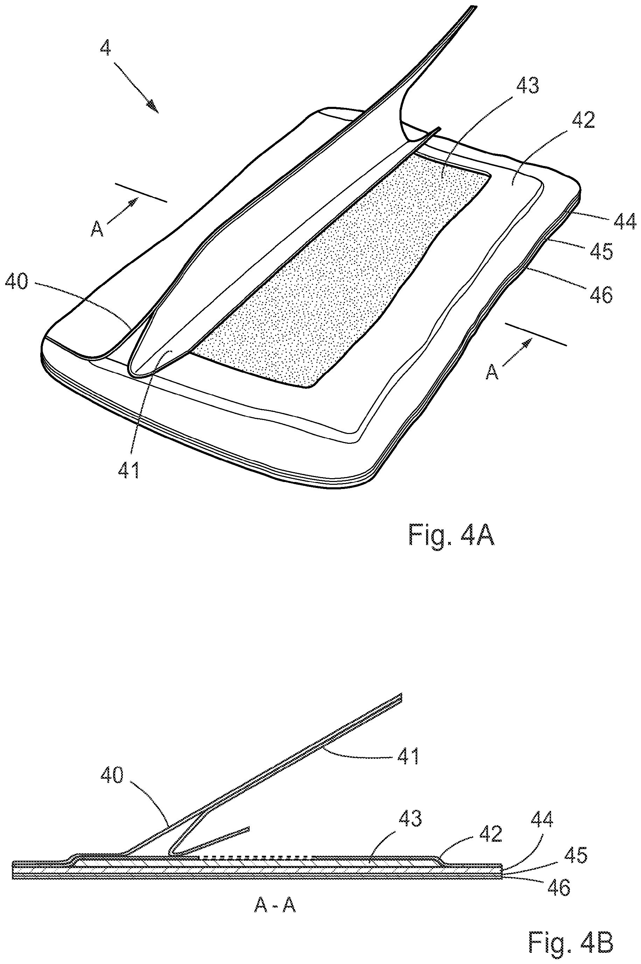

Fig. 4A showing embodiments being not part of the invention is a perspective view of a patch 4 having a closable backing layer 40, enabling fluid access to the patch interior during preparation of the patch 4 for use. Fig. 4B is a cross sectional view of the patch 4 illustrating the arrangement of the various layers and components of the patch 4 prior to application on the skin.

An access window to an absorbent core or reservoir 43 that is accessible from the exterior prior to use of the patch 4 may be provided. The access window is arranged in an intermediate layer 42 of the same material and characteristics as backing layer 40, i.e. NO impermeable and flexible. Upon use, either an activation fluid or an activated NO releasing fluid is introduced into the interior of patch 4, depending on if an NO donor compound is provided in the absorbent core or reservoir 43 or not. The closable backing layer is closed when NO release is initiated in the aforementioned way. A release liner 41 is arranged releasably on backing layer 40 and removed for adhesively closing the window by means of backing layer 40 being attached to intermediate layer 42. Thus an NO 35 impermeable arrangement is provided on the backing side of patch 4. Thereupon a second release liner 46 is removed from a skin contact enhancing layer 45, and the patch 4 is applied to the skin, whereupon NO is provided to the skin from the patch 4.

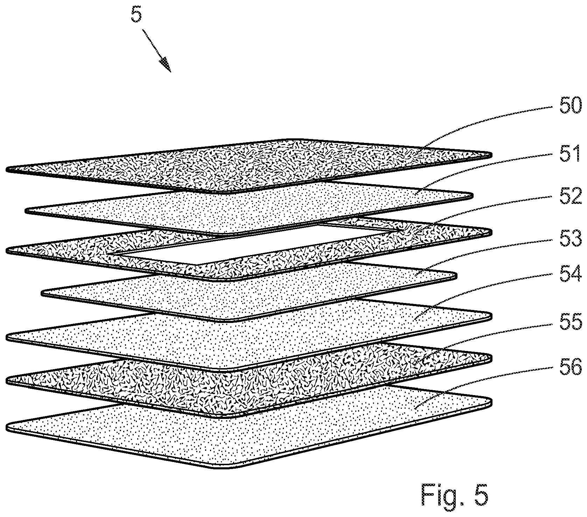

Fig. 5 showing embodiments being not part of the invention is a schematic illustration of the multi layered structure 5 of the embodiment of Fig. 4A. An NO impermeable backing lid 50 is arranged on top of the patch. A release liner 51 is arranged removably on backing lid 50. A windowed backing 52 is arranged on top of an absorbent layer 53, underneath which an NO permeable membrane 54 is arranged, followed by a skin contact enhancement layer 55 and a release liner 56.

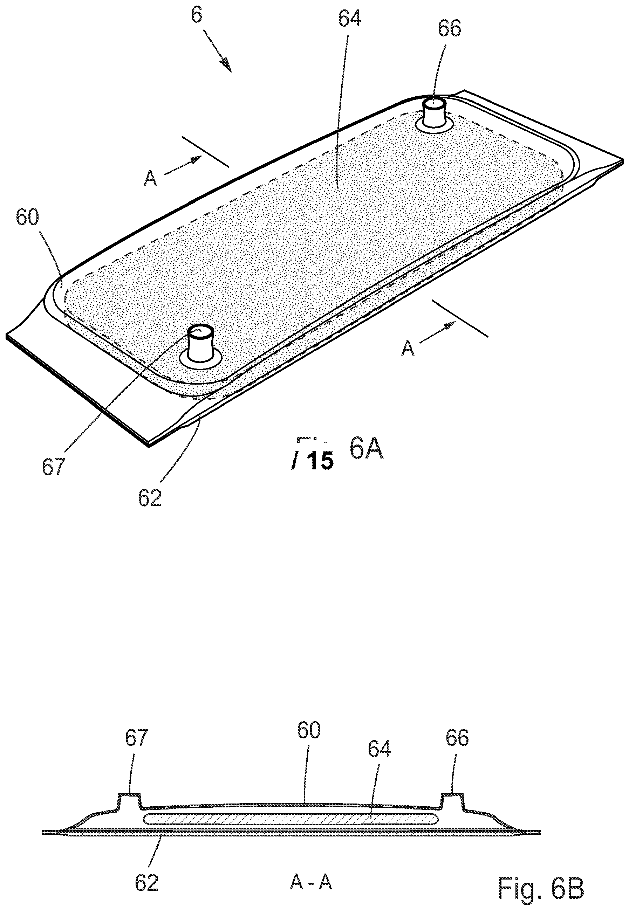

Fig. 6A showing embodiments being not part of the invention is a perspective view of a patch 6 having two access port ports 66, 67 allowing fluid communication with the interior of the patch 6. Fig. 6B is a cross sectional view of the patch shown in Fig. 6A. A backing layer 60 is impermeable for NO and a NO permeable layer 62 is arranged on the patient side of the patch 6. An absorbent core or reservoir 64 is arranged inside the patch, for receiving a fluid via port 66 and/or port 67. The absorbent core or reservoir may be made of an airlaid
material. Ports 66, 67 may be in form of a Luer lock system, e.g. for convenient connection of a syringe. A fluid guiding system may be arranged in, or adjacent to, the absorbent core or reservoir 64 for controlling distribution of the fluid in the patch 6.

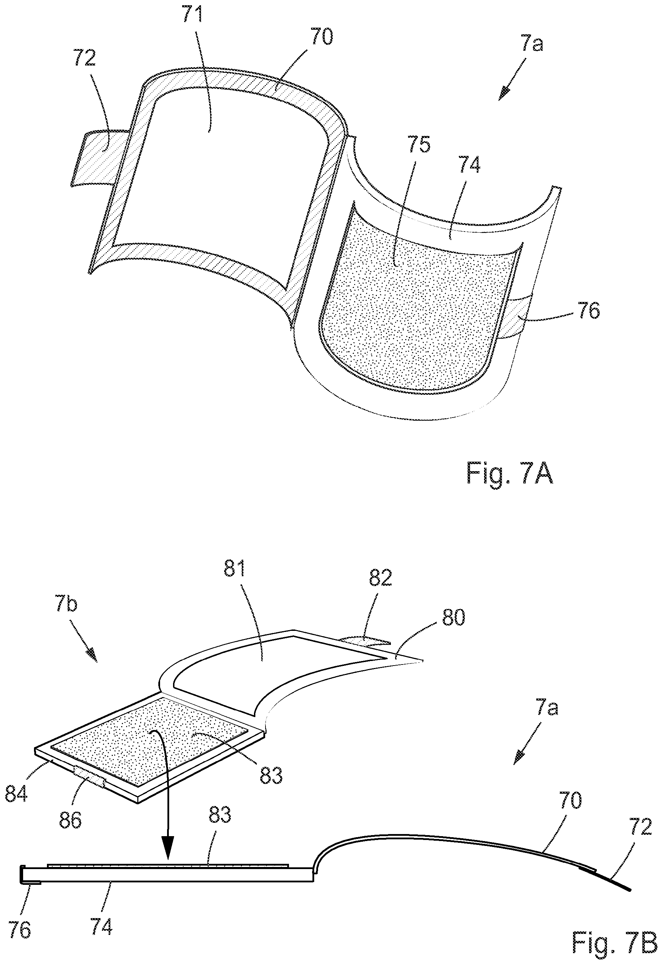

Fig. 7A showing embodiments being not part of the invention is a perspective view of a patch 7a of a patch system. Fig. 7B is a cross sectional view of the patch 7a as well as a further patch system component 7b comprising an exchange component of the patch system. The patch 7a comprises a sealingly repeatedly open- and closable backing enabling exchange of a patch system component during use. More precisely, is the patch 7a provided with a recess 75 into which an exchangeable absorbent core or reservoir 83 may be removably positioned, as illustrated in Fig. 7B. An NO impermeable backing layer 71 comprises a secure gas tight releasable adhesive 70 around its edge for mating with an edge 74 of or around a NO permeable layer. For further security and patient comfort and user friendliness, a loop and hook fastener 72, 76 is provided on the patch 7a. Patch system component 7b is a package in which an NO donor compound is provided in form of an exchangeable component 83. The package comprises a top layer 80. Top layer 80 is during storage in a gas tight manner attached to a bottom layer 84 in which the exchangeable component 83 is situated. Both top layer 80 and bottom layer 84 are NO impermeable and adapted for long term storage of the NO donor compound in exchangeable 10 component 83, e.g. gas tight, evacuated, and not transmissible for light. A closure system 82, 86 is provided on the packaging 7b. The exchangeable component 83 fits into the recess 75 in patch 7a. The exchangeable component 83 may be replaced in patch 7a with a fresh one, in order to recharge patch 7a with new NO donor. The release of NO from the NO donor is activated by adding fluid to the exchangeable component 83 either in the packaging 7b, or in the patch 7a. Then the backing lid is re-closed in a manner impermeable for NO, and the patch 7a 20 is ready for continued use.

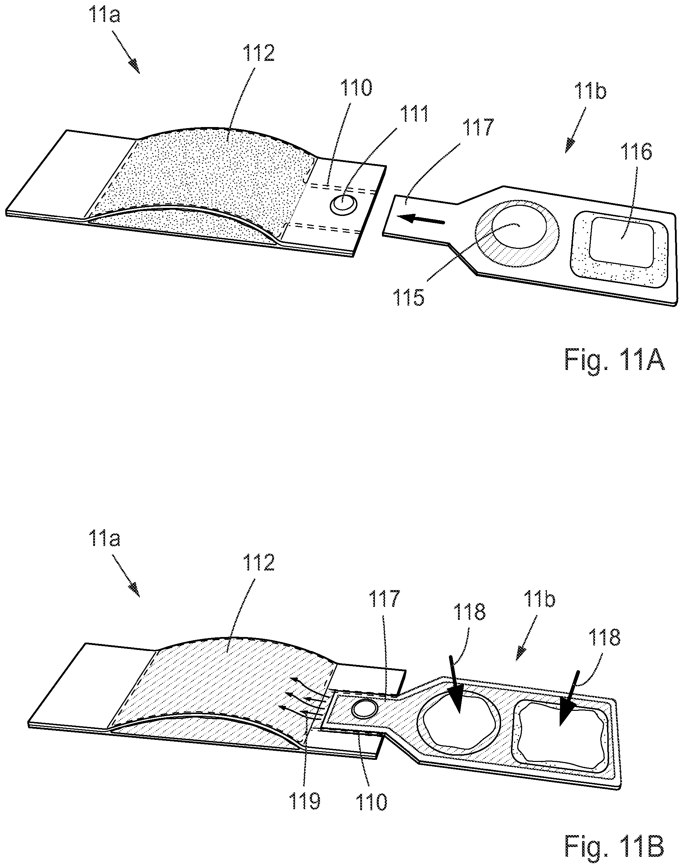

Fig. 8 showing embodiments being not part of the invention is a schematic illustration of a patch system component in form of a sealed bag 8, comprising an NO donor 88 in a breakable receptacle 87. By external force the receptacle 87, e.g. a glass vial, is broken and the NO donor is released into the surrounding activation fluid in the bag 8. Thus, the NO donor is activated and NO release is initiated. Via an outlet 89, the activated fluid may be applied to a patch, such as the patch shown in Fig. 4A or Fig. 11A.

Fig. 9 showing embodiments being not part of the invention is a schematic illustration of a patch system component comprising an NO donor component 92 and an activation fluid 91 in separate compartments of a multiple compartment device 9. Inside housing 90 of the device 9 the compartments may be broken, e.g. by external pressure on the compartments. The NO donor component 92 and activation fluid 91 are released from their compartments into a surrounding mixing chamber inside housing 90. Mixing may be enhanced by shaking the device 9. The fluid that thus is activated for NO release from the NO donor, may be transferred to a patch via an outlet port 93.

Fig. 10 showing embodiments being not part of the invention is a schematic illustration of a patch system component comprising an NO donor component 103 and an activation agent 101 in a multiple compartment arrangement of a bottle 10. The bottle is arranged such that the two compartments are twistable against each other, where upon a seal between the two compartments is broken and the NO donor component 103 and the activation agent 101 get into fluid contact in the bottle 10. Mixing may be enhanced by shaking the bottle 10. The fluid that thus is activated for NO release from the NO donor, may be transferred from the bottle to a patch via an outlet port 102.

Fig. 11A showing embodiments being not part of the invention is a perspective view of a patch system comprising a patch 11a and the multiple compartment device 11b of the type illustrated in Fig. 9, prior to admixing the NO donor component and the release activation fluid from two compartments 115, 116. The arrow at the outlet port 117 illustrates how the multiple compartment device 11b may be joined with an inlet port 110 of the patch 11a, as illustrated in Fig. 11B. Fig. 11B is a perspective view of the patch system of Fig. 11A after admixing the NO donor component and the release activation fluid in multiple compartment device 11b, as explained above. The activated NO releasing fluid is introduced into the patch and absorbent core or reservoir 112 via outlet port 117 of the multiple compartment device 11b and inlet port 110 of the patch 11a, wherein the flow is illustrated by arrows 119. During filling, leakage may be reduced or avoided by a valve 111, schematically illustrated in Figs. 11A and 11B. When the activated fluid is transferred to the patch 11a, the multiple compartment device 11b is removed, valve 111 is closed and the patch 11a is ready for application to the patient.

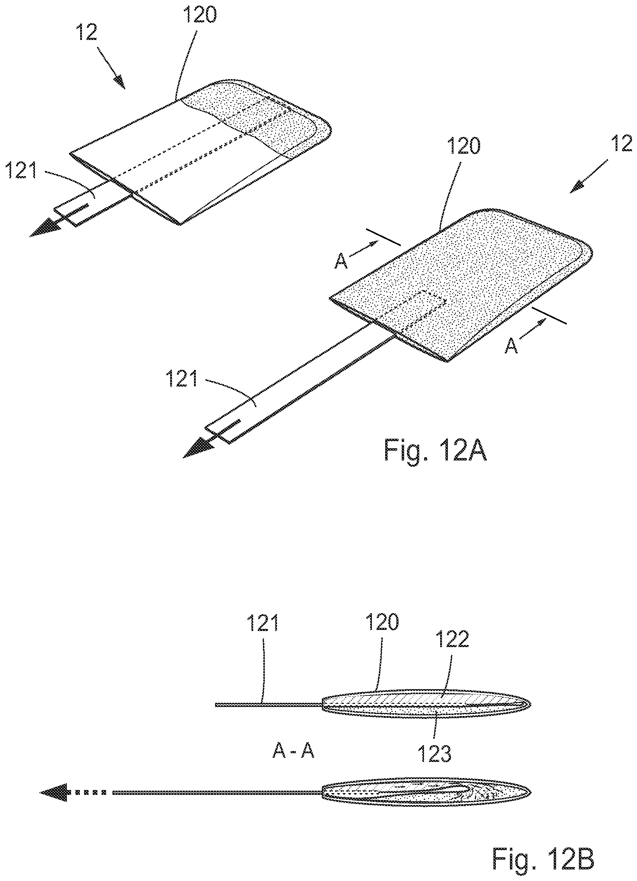

Fig. 12A is a perspective view of a patch 12 according to the invention comprising an NO donor component and an activation agent in a multiple compartment arrangement. Fig. 12B is a cross sectional view of the patch 12 shown in Fig. 12A along a line A-A. The patch 12 comprises a first compartment 122 and a second compartment 123, divided by an impermeable film, e.g. of aluminum foil. The first compartment 122 contains an NO donor compound and the second compartment 123 contains an activation fluid. The impermeable film may be removed between the compartments, as illustrated, by drawing a strip 121 attached to the impermeable film. In this manner, activation is easily accomplished for the user and the patch 12 is ready for application.

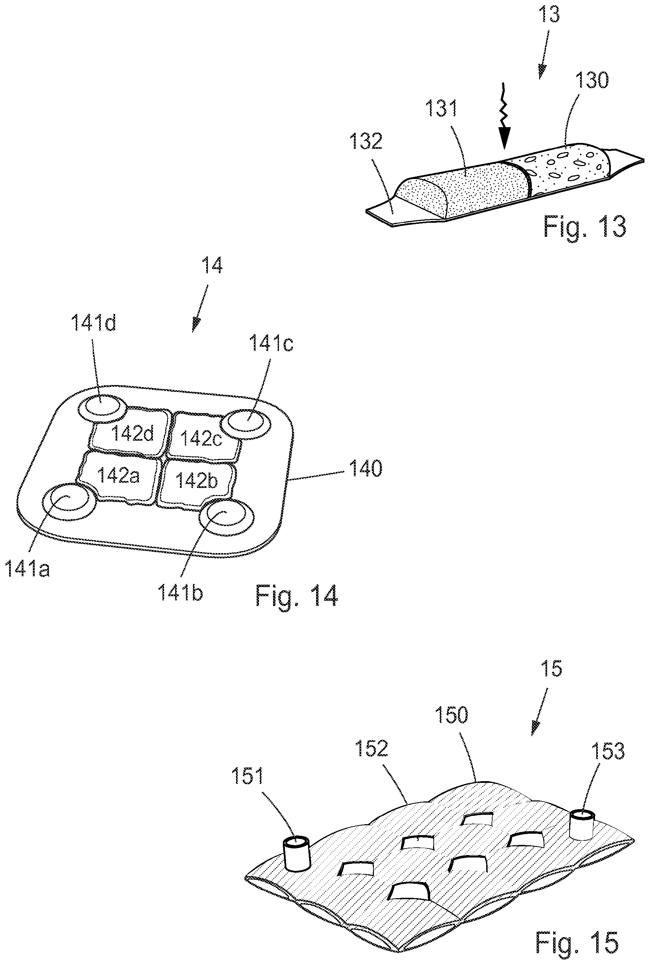

Fig. 13 showing embodiments being not part of the invention is a perspective view of a further patch 13 comprising an NO donor component 131 and an activation agent 130 in a breakable multiple compartment arrangement 132. The two compartments may be connected to each other by breaking a seal that is arranged in-between by external force. A NO releasing fluid may thus be activated, wherein the patch 13 may also be shaken for intensely mixing the two components. A release line is then removed from an NO permeable membrane of patch 13 and the patch is ready for application.

Fig. 14 showing embodiments being not part of the invention is a perspective view of a patch 14 having multiple compartments, subdividing the active area of the patch into different treatment zones corresponding to different compartments 142a-d. Each of compartments 142a-d is divided from the other compartment and accessible via a fluid communication port 141a-d, respectively. In this way the active usage time of the patch may be prolonged my sequentially filling the compartments with a NO releasing fluid or an activation fluid, depending on whether a compartment 142a-d contains an NO donor compound or not. The compartments may be geometrically arranged in a different way than illustrated, e.g. interleaved such that the treatment area is covered by each of the compartments. By means of this embodiment, the treatment time may be enhanced by activating the compartments subsequently when the useful amount of NO released from the NO donor compound decreases to a predefined threshold. This threshold may be indicated by the patch itself, e.g. by changing color at the surface of the compartment where NO release is below the threshold. The patch may comprise an active indicator on top of the patch, in order to indicate to the user that the NO has been utilized.

Fig. 15 showing embodiments being not part of the invention is a perspective view of a patch 15 having passageways 152 for a fluid to and from the skin to which the patch 15 is applied in use. The patch is built up of adjacent compartments 150 fluidly connected to each other and to two ports 151, 153. The patch 15 is filled with NO releasing fluid or activated as described above. When applied to a wound, the patch 15 allows for passage of exudate through the passageways 152, e.g. to an exchangeable absorbent applied on the back of patch 15. In some embodiments, such as in the embodiment illustrated in Fig. 16, the NO donor is integrated into the absorbent (layer 200). All matter that may activate a release of NO from the NO donor compound, such as air, oxygen, in the pouch is extracted from the pouch during manufacturing thereof. This is accomplished through drawing sub atmospheric pressure in the absorbent during assembly of the device.

The activation fluid is kept in a pouch on top of the absorbent. The pouch is non permeable to NO and non permeable to water. For activation of the device the pouch is squeezed and will break open into the evacuated absorbent. The liquid is distributed in the absorbent promoted by the sub atmospheric pressure. Thus a quick and advantageous homogenous distribution of the activation fluid is obtained in the absorbent. The incorporated NO donor in the absorbent will start to release NO when it comes in contact with the liquid. The released NO permeates through the membrane towards the topical site to be treated. Since the top layer is substantially impermeable to NO, the release will efficiently be directed towards the treatment site.

In more detail, the embodiment illustrated in Fig. 16 showing embodiments being not part of the invention is a topical treatment patch 16. The patch 16 comprises a pouch for filling with a liquid activation agent. The pouch comprises a pouch top 162 and a pouch bottom 163. The pouch top 162 and the pouch bottom 163 are sealingly attached to each other with backing layer 161 in between - in two steps 162-161 with a strong sealing 161-163 with a weaker sealing compared to the former or in one step 162-161-163 depending on rupturing mechanism. At edge areas 162a and 163a, for instance by ultrasonic welding achieving a sealed engagement around the edges 162a, 163a. In an embodiment, the pouch may comprise a small opening defined by 1620 and 1621, wherein the edge is not sealed in the area of the openings. The second opening 1621 may be shorter than 1620 which during welding 1621 may be done closing the opening 1621 while leaving 1620 open. Through the opening 162 filling of activation fluid may take place. After fluid filling the opening 1620 is closed and a sealed pouch filled with activation fluid is provided. The material of the pouch top 162 and the pouch bottom 163 is non permeable to both NO and fluid. In this manner the pouch containing fluid and thus the patch 16 may be stored over a long time.

As layers 163, 162 and layer 161, respectively, may be produced from different materials, this may have several advantages. For instance, varying flexibility of the patch may be provided. Layers 163, 162 need to be more long term non-permeable than layer 161. Layer 161 only needs to be substantially non-permeably for NO for a substantially shorter time than pouch layers 163, 162.

The patch 16 further comprises a backing 161 that is non permeable for NO. The backing 161 is flexible, e.g. a flexible film, to ensure flexibility of the patch 16. Backing layer 16 is sealed together with layer 166, e.g. by ultrasonic sealing. The seal is performed in a sub atmospheric pressure environment.

The liquid pouch bottom 163 is ultrasonic sealed around the edges, optionally except for a small opening corresponding to opening 1620 explained above. The opening avoids that the top opening 1620 is sealed. The seal at the edges is provided as releasable. It may be provided such that it breaks upon pressure, or by alternative features as described below.

An absorbent layer 164 is devised to absorb and retain the activation liquid so that it is well distributed over the surface of the layer, even when applying deformation and point pressure on the patch. Absorbent layer 164 also stores or is integrated with the NO donor.

An adhesive layer 165 is provided to ensure a good contact between the absorbent layer 164 and the membrane 166. In an embodiment the adhesive layer is thus integrated with the absorbent layer. In such an embodiment the NO donor may be arranged in the adhesive layer 165 and still be integrated with the absorbent layer 164.

The membrane 166 is devised to regulate NO transfer to the topical site. It may be devised to keep fluid and NO donor rests in the patch, e.g. in dependence of the toxicity thereof, as explained above.

Further, the patch 16 comprises a contact enhancement layer 167, and a release liner 168. The release liner 168 is devised to have a low gas permeability in order to ensure that the sub atmospheric pressure is kept in the absorbent layer 164, since the membrane 166 is permeable to air. An outer skin adhesive 169 is provided to keep the patch 16 in place when applied to the treatment site of the patient. The contact enhancement layer 167 may comprise adhesive in order to not harm, injury or damage the treated area and therefore does not have enough strength to keep the patch 16 in place at the treatment site. The adhesive 169 may advantageously be substantially impermeable to NO, thus further enhancing the directional application of NO during use of the patch 16. The adhesive force of adhesive 169 is stronger than that of contact enhancement layer 167 assuring firm fixation of the patch. This may also advantageously contribute to the direction of NO towards the treatment site as direction is more effective by this arrangement.

The NO donor material may be distributed in the absorbent material 164 though the following techniques:
- multiple injections to have it homogenously spread
- by spraying over the surface of the absorbent layer 164 prior to assembly into patch 16
- Screen printing techniques providing advantageous distribution patterns
- Integration into the adhesive layer 165

The rupturing of the fluid pouch may be performed in different ways:
a weak seal towards the evacuated absorbent 164, i.e. the seal is preserved during packaging, transport and storage of the patch 16, but may at least partly be removed by an external influence, such as a force leading to rupturing, breaking, or releasing the seal towards the evacuated absorbent 164:
   - The seal may be a weak heat-seal
   - The seal may be a weak adhesive
   - The seal may have a shape that directs the force towards specific areas to initiate rupture of the seal, e.g. indents 1640 in the seal area 163a
   - The liquid top may have a shape that directs the force towards specific areas to initiate rupture of the seal, e.g.
   - Local weakness of the pouch material, e.g. in the pouch along the weld generated during the pouch welding. Elongation of the film from a folded position to a flat position generated from manufacturing the same
   - Alternatively or in addition the pouch material may have varying thickness, defining one or more areas of locally reduced structural strength in the pouch material. That means that an attenuation may be provided in the film towards evacuated absorbent
   - a sharp needle that is arranged to penetrate the film towards the evacuated absorbent layer 164 upon activation

In addition or alternatively, the patch 16 may comprise one or more of the following advantageous features:
- Slits 164 in the absorbent layer 164 may be arranged to increase flexibility of the patch 16 as well as enhancing fluid distribution in the generated channels and to be continuous or non-continuous along the longitudinal extension
- An adhesive layer 165 between the absorbent layer and the membrane 166 to facilitate good contact
- Fixation of the backing layer 161 to the absorbent to reduce any void volume
- An extra strong adhesive layer 169 arranged around the treatment area of the patch 16.

Encapsulation of unwanted species in the patch may be provided accordingly to the above described dependent choice of permeability of membrane 166.

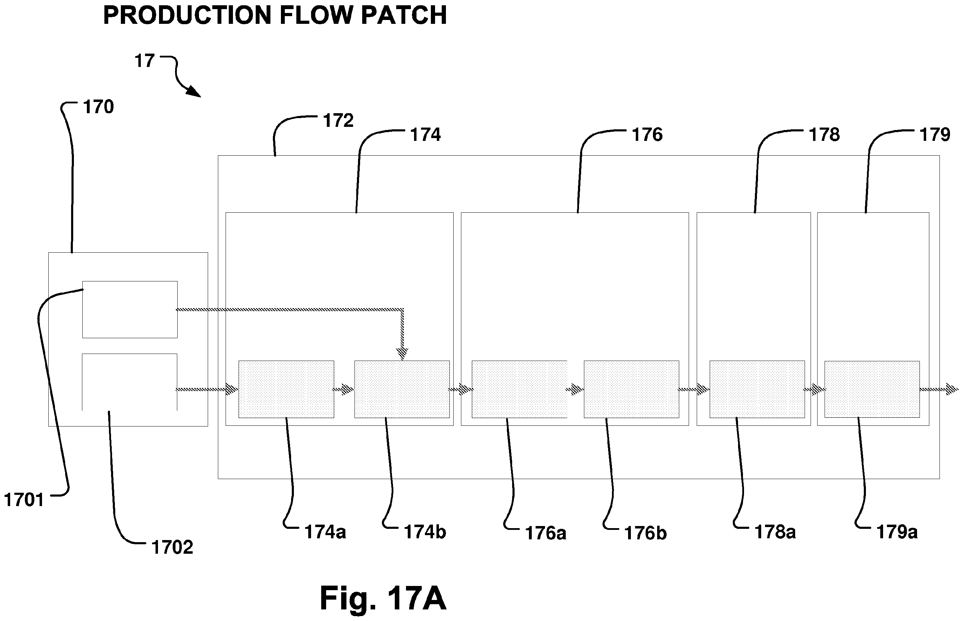

In an embodiment, the Production flow 17 of a patch consists of the following steps, as illustrated Fig 17a;

Film converting 170: The different product components, i.e. the top part comprising the fluid compartment and the bottom part comprising the skin adhesive, membrane and the absorbent. These parts are diecut out of film rolls and lamination of different layers is made during the converting operation. As a secondary operation the fluid pouch are welded 1701 in either a one step operation or a two step operation depending of the weld be used as a mean of an opening of the fluid during the activation.

Assembly fluid filling 172: On the assembly with the absorbent exposed the donor is applied by spraying or injection 174a. Directly thereafter the second assembly with the pouch is applied on top and in a sub atmospheric pressure chamber the two assemblies are welded together creating an oxygen free and sub atmospheric pressurized contained absorbent 174b. The fluid pouch is then filled 176a and sealed 176b.

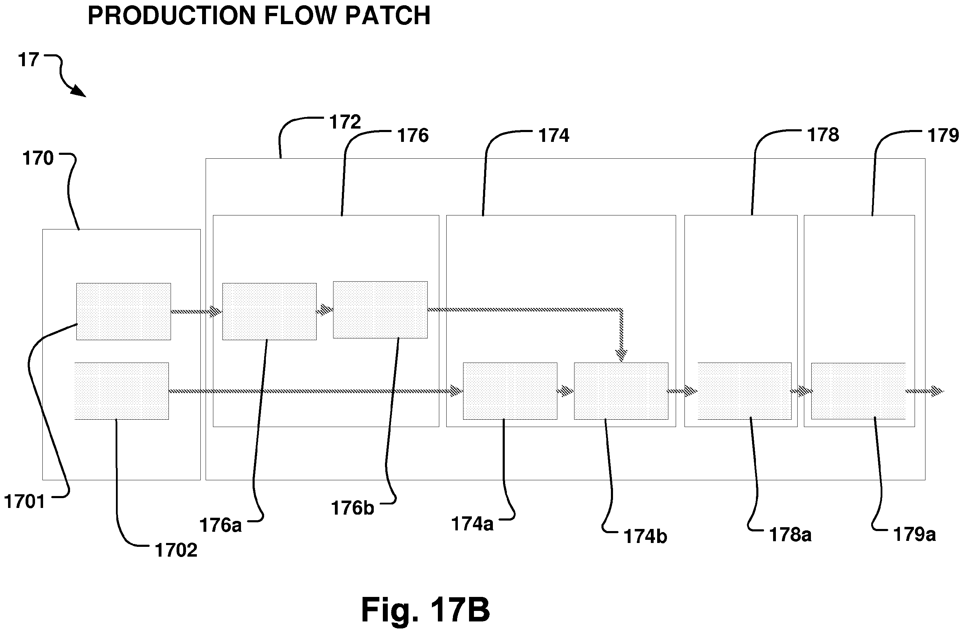

Alternatively, Fig. 17b, the assembly fluid filling 172 could be achieved as the first assembly the pouch is filled 176a and sealed forming a film with filled pouches176b. On the assembly with the exposed absorbent the donor is first applied by spraying or injection 174a. Directly thereafter the first assembly with the pouch is applied on top and in a sub atmospheric pressure chamber the two assemblies are welded together creating an oxygen free and sub atmospheric pressurized contained absorbent 174b.

The now complete product, by either alternative path, is then package into a primary package 178 and welded under sub atmospheric pressure 178a to assure an oxygen free environment and to further assure that the sub atmospheric pressure around the absorbent is assured. This may be omitted if e.g. a long-term non-permeable release layer is attached to backing.

Further, production flow 179 may comprise final packaging as assemble product box, sterilization or other necessities or demands 179a.

A product box may be omitted in embodiments where a long-term release liner is attached to the backing layer, thus effectively preventing any leakage into or from the patch during storage.

### Fluid system

Addition of fluids to the topical patch system may for instance be done in the following manners:
- By having a closable lid in the top cover through which fluids may be poured into the patch. The lid is then closed and the device can be attached to the skin, see Figs. 4A and 4B.
- By adding a one directional valve through which the fluids may be inserted into the patch pouch, see Figs. 6A and 6B. In the case of integrating the NO donor compound in the pouch, all matter that may activate a release of NO from the NO donor compound, such as moisture, air, oxygen, in the pouch is extracted from the pouch during manufacturing thereof. Optionally, the pouch may be filled with an inert matter. After storage, and for activation of the patch, an activation fluid is injected through the valve into the pouch and absorbed into the absorbent core, whereupon NO release is activated and the patch is "active" for medical or cosmetical treatment. In order for the fluid to be spread evenly over the area a wicking layer may be advantageously provided.
- By having an integrated first reservoir for the NO donor in the patch, which is arranged in a sealed manner apart from the fluid. The fluid may be integrated together with the absorbent core or in a separate second reservoir. Prior to use the first and perhaps the second reservoir are manipulated to open, e.g. by squeezing the reservoir(s) to break. The NO donor spreads and comes in contact with the activation fluid and the NO release starts.
- By adding a pH adjusting feature to the absorbent core, or in that the absorbent core itself has this capability, the fluid to be mixed with the donor may be inactive and only when the donor fluid comes in contact with the absorbent core the system is balanced to a suitable pH and the NO release is initiated.

For mixing of fluids, i.e. mixing of the activation fluid with the NO donor compound that for instance is provided in powder form, a number of embodiments are now in described. Mixing is performed in such a manner that the NO donor compound is spread evenly in order to provide an efficient controlled release of NO from the patch. Various embodiments comprise, amongst others:
- The NO donor compound is provided in dry form and a pre dissolving fluid is mixed with the NO donor compound separately from the patch in order to dissolve the NO donor efficiently and to provide a pre dissolved NO donor fluid.
   ∘ The pre dissolved NO donor fluid is added to the patch, wherein a component inside the patch, e.g. the absorbent core, is soaked with a NO release activation fluid. Thereby an effective, controlled release of NO is activated in the patch.
   ∘ Alternatively, the pre dissolved NO donor fluid is added to a NO release activation fluid, or vice versa, whereby a NO releasing fluid is provided. The NO releasing fluid is added to the patch, i.e. to a component inside the patch, e.g. the absorbent core, which in this manner is soaked with the NO releasing fluid is. Thereby an effective, controlled release of NO is provided from the patch.
- The NO donor compound and solid activation components are provided in dry form in the patch, e.g. as fibers or a powder incorporated into the absorbent core. A fluid is added to the dry patch upon use of the patch, resulting in the solid activation components dissolving in the fluid and thus activating the release of NO from the NO donor compound.
- The NO donor compound is provided in dry form and mixed with a fluid comprising a pre dissolving and activation fluid, or only an activation fluid. The mixture, in which NO release from the NO donor compound is activated, is add to patch for providing NO release from the patch.

### Further features of the topical patch

Some embodiments of the topical patch have enhanced functionality by comprising features, such as:
- An exchangeable absorbent material is provided, such that the layer towards the skin to be treated is stable during treatment and does not need to be replaced. This is an important feature in specific conditions within the area of wound care treatment.
- A wicking system for ease of spreading the fluid over the absorbent core is provided in the patch. Such a wicking system may for instance be provided in the form of a layer that is configured to spread a fluid in the patch before it passes into the absorbent.
- The patch may be provided in the form of a fluid pouch with a valve controlling fluid direction into the pouch and allow displaced gas to egress from the pouch.
- The pouch may be provided with different sections to assure that an even distribution of the fluid is obtained. In addition different treatment zones within the patch maybe provided by the different sections.
- The absorbent core may be made of various materials adapted to absorb fluid and contain it. Examples for such materials are super absorbing materials, for instance comprising SAP and/or SAF in it, or a hydrophilic foam, or a hydro gel.

### Examples:

### Example 1: Intense dose patch

The patch has a high NO dose level such as between 0,01-100pmol/(cm2*min), such as 0,1 pmol/(cm2*min). It has a short duration of use, approximately ten minutes up to two hours, such as 1 hour use. To accomplish this intensity and lengths in dose curve a sufficient amount of NO donor has to be used such as 0,01-10 mg/cm2, such as 0,1 mg/cm2. Also a low pH in the activation fluid is needed, less than pH 6, such as pH 5. The volume of buffer needed is dependant of the NO donor amount used. For a 0,1 mg NO donor/cm2 patch, it is necessary to have 0,1-0,4 mL/cm2 depending on buffer strength. To ensure sufficient contact with skin a contact enhancement layer is used.

### Example 2: Extended dose patch

The patch have a medium NO dose level such as between 0,001-10pmol/(cm2*min), such as 0,05 pmol/(cm2*min). It has an extended duration of use of six to twelve hours, such as eight hour use. To accomplish this intensity and length in dose curve, a sufficient amount of NO donor has to be used, such as 0,01-10 mg/cm2, such as 0,2 mg/cm2. In addition, a neutral to slightly acidic pH in the activation fluid is needed, such as pH 7. The volume of buffer needed is dependant of the NO donor amount used. For a 0,2 mg NO donor/cm2 patch it is necessary to have 0,2-0,8 mL/cm2, depending on buffer strength. To ensure sufficient contact with skin a contact enhancement layer is used.

Treatments and therapies that the patch is advantageous for are for instance:
treatment of peripheral neuropathy;
treatment of diabetic ulcers;
wound care, such as post operative healing support;
infection prevention and treatment;
cosmetic use, including scar reduction, skin lifting etc. ;
antifungal treatment, for instance of onychomycosis or mixed infections;
anti virus treatment, e.g. for wart treatment.

The patch is suitable for human or for veterinary medicine.

A kit may comprise several components according to the above description in an advantageous manner.

The patch may be regarded a medicament according to certain embodiments.

A use of nitric oxide may be provided for manufacturing a patch according to some embodiments.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present. As used herein, the term "and/or" includes any and all combinations of one or more of the associated listed items.

The present invention has been described above with reference to specific embodiments. However, other embodiments than the above described are equally possible within the scope of the invention. Different method steps than those described above, performing the method by hardware or software, may be provided within the scope of the invention. The scope of the invention is only limited by the appended patent claims.

## Claims

1. A topical nitric oxide delivery device for arrangement at a treatment site, comprising
a nitric oxide releasing fluid in use of said device, wherein said nitric oxide releasing fluid comprises an activation fluid for activation of a release of nitric oxide from a nitric oxide donor compound in use of said device, said nitric oxide donor compound being comprised in said nitric oxide delivery device;
said nitric oxide donor compound and said activation fluid are separated from each other in separate reservoirs by an impermeable film in said device during storage of said device and arranged to be brought into contact by removal of the impermeable film for said release of nitric oxide from said nitric oxide releasing fluid, wherein said nitric oxide releasing fluid is at least partly liquid;
a reservoir (200) arranged to retain said nitric oxide releasing fluid; and
a nitric oxide permeable layer (300) adapted to contact said treatment site and adapted to provide an interfacial area for efficient transport of said nitric oxide from said nitric oxide releasing fluid to said treatment site; wherein at least said nitric oxide permeable layer is geometrically flexible such that a loss of said interfacial area between said nitric oxide permeable layer and said treatment area is avoided in use of said device,
whereby said device is adapted to provide a desired, controllable and consistent delivery of said nitric oxide even under physical deformation of said device from said reservoir to said treatment site while avoiding transport of undesired species from said reservoir to said treatment site.

2. The device according to claim 1, wherein the impermeable film is attached to a strip (121) for removal of the impermeable film between the compartments by drawing said strip (121).

3. The device according to claim 1 or 2, wherein said nitric oxide permeable layer (300) further comprises a skin contact enhancement layer that is adapted to be arranged adjacent to a skin contact area at said treatment site, or has inherent skin contact enhancement properties, that is permeable for said nitric oxide, such that it provides sufficient skin contact upon use thereof, and wherein said skin contact enhancement layer is an adhesive layer, such as a silicone gel layer, a pressure sensitive adhesive (PSA), or a hydrogel layer, that thereby is adapted to enhance contact between the said nitric oxide permeable layer (300) and said skin area upon use.

4. The device according to claim 3, wherein said skin contact enhancement layer is configured to fill out unevenness in the skin, such that leakage of nitric oxide away from said nitric oxide delivery device instead of into the skin is prevented.

5. The device according to claim 3 or 4, wherein said skin contact enhancement layer provides enough adhesion capabilities to stick said nitric oxide delivery device to the skin.

6. The device according to any of claims 3 to 5, wherein said skin contact enhancement layer is attached to the NO permeable layer on the side that in use is turned towards the skin.

7. The device according to any of the preceding claims, wherein said nitric oxide permeable layer (300) is adapted to barrier transport of said undesired species from said reservoir to said treatment site by means of size or solubility selectivity thereof.

8. The device according to any of the preceding claims, wherein said reservoir (200) comprises a solid media, and wherein said device further comprising a substantially nitric oxide impermeable backing layer adapted to provide an increased partial pressure of gaseous nitric oxide and consequently a concentration of said nitric oxide in said nitric oxide releasing fluid distributed in said solid media contained in said reservoir in contact with said nitric oxide permeable layer which is permeable with respect to nitric oxide substantially in the direction of the treatment area.

9. The device according to claim 8, wherein said nitric oxide releasing fluid is evenly distributed in said solid media with high affinity for said nitric oxide releasing fluid, such that a high interfacial area between said nitric oxide releasing fluid and said nitric oxide permeable layer is provided under environmental stress of said device, such as bending, applying uneven pressure to the device, and tilting the device.

10. The topical nitric oxide delivery device according to any of the preceding claims, comprising
a backing layer (100) substantially impermeable to nitric oxide, arranged and configured to limit a loss of nitric oxide concentration during release of nitric oxide from said nitric oxide releasing fluid in the delivery device, wherein the nitric oxide permeable layer (300) in use of the delivery device is oriented towards an area to be treated, and wherein said reservoir is an absorbing layer (200) arranged between said backing layer (100) and said nitric oxide permeable layer (300), the absorbing layer is arranged to retain said nitric oxide releasing fluid therein in such a manner that nitric oxide released from said nitric oxide releasing fluid is directed within the delivery device and through the nitric oxide permeable layer (300) towards a skin area by an increase of partial pressure or concentration of nitric oxide therein.

11. The topical nitric oxide delivery device according to any of the preceding claims, wherein said reservoir is an absorbing layer (200) that comprises a gel based absorbing system of a gelling material, configured to absorb and retain said a nitric oxide releasing fluid.

12. The device according to any of the preceding claims, wherein said reservoir is a fluid reservoir comprised in an absorbing layer (200) that provides storage and entrapment for said nitric oxide releasing fluid and a nitric oxide donor therein in a fluid stage.

13. The device according to any of the preceding claims, wherein said nitric oxide permeable layer (300) is a membrane selectively permeable for gaseous nitric oxide or dissolved nitric oxide, whereby nitric oxide released inside the topical nitric oxide delivery device is directed towards said treatment site.

14. The topical nitric oxide delivery device according to claim 12, wherein said fluid reservoir is a gelling material that contains the nitric oxide releasing fluid within its boundaries.

15. The device according to claims 11 to 14, wherein said absorbing layer (200) is fully wettened, and wherein said absorbing layer (200) comprises a fluid content of more than 60%, such as 80% or more than 90%.

16. The topical nitric oxide delivery device according to any of the preceding claims, wherein said NO permeable layer (300) is configured to regulate the NO transfer speed from the device to the dermal treatment site, wherein said nitric oxide permeable layer (300) is a membrane of nonwoven or porous materials such that said nitric oxide permeable layer (300) is configured for transport of a fluid having NO dissolved therein and configured for good communication across said nitric oxide permeable layer (300).

17. The device according to any of the preceding claims,
wherein said nitric oxide permeable layer (300) is made of a high moisture vapour transfer rate (MVTR) material, such as a micro porous membrane or a hydrophilic membrane composed of a partially hydrophilic block co-polymer membrane such that said nitric oxide permeable layer (300) is configured for transport of a fluid having NO dissolved therein and other nontoxic nonirritant low molecular weight substances across the membrane but retain larger molecules e.g. the NO generating system,
configured for medium communication across said nitric oxide permeable layer (300).

18. The device according to any of the preceding claims,
wherein said nitric oxide permeable layer (300) is made of a medium or low MVTR material such that said nitric oxide permeable layer (300) is configured for transport of a fluid having NO dissolved therein and configured for low communication across said nitric oxide permeable layer (300).

19. The device according to claim 1 to 17, wherein,
said nitric oxide permeable layer (300) is chosen such that it is highly selective towards NO transport, e.g. operating via a size selectivity mechanism, allowing no liquid communication across the membrane.

20. The topical nitric oxide delivery device according to any preceding claim comprising an adhesive layer (169) around a treatment area of said topical nitric oxide delivery device, wherein said nitric oxide permeable layer is arranged within the boundaries of said adhesive layer (169) and wherein said adhesive layer is more adhesive than said towards said treatment site than said nitric oxide permeable layer.

21. The topical nitric oxide delivery device according to any preceding claim , comprising a fluid direction system including an nitric oxide donor compound, arranged to receive the activation fluid and direct the activation fluid to said nitric oxide donor compound, such that said activation fluid in use dissolves said nitric oxide donor compound prior to applying it into the absorbent layer (200), wherein said fluid direction system is integrated with said reservoir.

22. Use of nitric oxide in the manufacture of a device according to any of claims 1 to 21.

23. A method of cosmetic treatment comprising delivering nitric oxide, said method comprising:
providing a nitric oxide releasing fluid and activating nitric oxide release from said nitric oxide releasing fluid in a nitric oxide delivery device according to any of claims 1-21;
placing the activated nitric oxide delivery device in contact with a skin area of a body, with a nitric oxide permeable layer (300) of the delivery device oriented towards said skin area, wherein a permeability of said nitric oxide permeable layer (300) is selectively chosen 20 depending on a toxicity level of said nitric oxide fluid; and
delivering nitric oxide topically from said nitric oxide delivery device to said skin area via said nitric oxide permeable layer (300) by
a backing layer (100) limiting a loss of nitric oxide concentration during release of nitric oxide from said nitric oxide releasing fluid; and
an absorbing layer (200) between said backing layer (100) and said nitric oxide permeable layer (300) retaining said nitric oxide releasing fluid therein; thereby increasing a partial pressure or concentration of nitric oxide in said nitric oxide releasing fluid and
directing nitric oxide from said nitric oxide releasing fluid within said delivery device and through said nitric oxide permeable layer (300) towards said skin area, and
releasing said nitric oxide from said nitric oxide donor system such that said partial pressure and thus a concentration of dissolved nitric oxide in said nitric oxide delivery device increases for said delivering of nitric oxide, wherein said skin area is a cosmetic treatment site, such as a site of scars to be reduced by aid nitric oxide, or a site of sagged skin or wrinkles to be lifted by said nitric oxide.

24. The method according to claim 23, comprising fixating said nitric oxide delivery device to said skin area by means of a skin contact enhancement layer permeable for nitric oxide.

25. A method of manufacturing a topical delivery device according to any of claims 1 to 21, comprising film converting (170), and assembly fluid filling (172) in a sub-atmospheric pressurized environment, or assembly fluid filling (172) by filling and sealing a fluid container and arranging the filled container in a sub atmospheric pressure environment, thus creating an oxygen free and sub atmospheric pressurized contained reservoir arranged to retain said nitric oxide releasing fluid.

26. A method of manufacturing a topical delivery device according to claim 25, wherein said film converting comprising
die-cutting a skin adhesive, a membrane and an absorbent from film rolls for a bottom part of the topical delivery device,
die-cutting a fluid compartment from film rolls for a top part of the topical delivery device,
welding a fluid pouch, and wherein said assembly fluid filling comprising applying a nitric oxide donor compound by spraying or injection to said absorbent,
applying the fluid pouch on the top part of the topical delivery device, welding the fluid pouch to the bottom part of the topical delivery device in a sub atmospheric pressure chamber creating said oxygen free and sub atmospheric pressurized contained absorbent, and
filling and sealing said fluid pouch.

## Patentansprüche

1. Topische Stickoxid-Abgabevorrichtung zur Anordnung an einer Behandlungsstelle, umfassend
ein Stickoxid freisetzendes Fluid bei Verwendung der Vorrichtung, wobei das Stickoxid freisetzende Fluid ein Aktivierungsfluid zum Aktivieren einer Freisetzung von Stickoxid von einer Stickoxiddonorverbindung bei Verwendung der Vorrichtung umfasst, wobei die Stickoxiddonorverbindung in der Stickoxid-Abgabevorrichtung umfasst ist;
die Stickoxiddonorverbindung und das Aktivierungsfluid, die voneinander in getrennten Behältern durch einen undurchlässigen Film in der Vorrichtung während der Lagerung der Vorrichtung getrennt sind und angeordnet sind, um in Kontakt gebracht zu werden, indem der undurchlässige Film für die Freisetzung von Stickoxid aus dem Stickoxid freisetzenden Fluid entfernt wird, wobei das Stickoxid freisetzende Fluid zumindest teilweise flüssig ist;
ein Behälter (200), der angeordnet ist, um das Stickoxid freisetzende Fluid zurückzuhalten; und
eine Stickoxid durchlässige Schicht (300), die so angepasst ist, dass sie die die Behandlungsstelle berührt und angepasst ist, um einen Grenzflächenbereich für einen effizienten Transport des Stickoxids von dem Stickoxid-Freisetzungsfluid zu der Behandlungsstelle bereitzustellen; wobei mindestens die Stickoxid durchlässige Schicht geometrisch flexibel ist, so dass ein Verlust der Grenzfläche zwischen der Stickoxid durchlässigen Schicht und dem Behandlungsbereich bei der Verwendung der Vorrichtung vermieden wird,
wobei die Vorrichtung angepasst ist, um eine gewünschte, kontrollierbare und gleichmäßige Abgabe des Stickoxids selbst unter physikalischer Deformation der Vorrichtung von dem Behälter zu der Behandlungsstelle bereitzustellen, während der Transport von unerwünschten Spezies von dem Behälter zu der Behandlungsstelle vermieden wird.

2. Vorrichtung nach Anspruch 1, wobei der undurchlässige Film an einem Streifen (121) zum Entfernen des undurchlässigen Films zwischen den Kompartimenten durch Ziehen des Streifens (121) befestigt ist.

3. Vorrichtung nach Anspruch 1 oder 2, bei der die Stickoxid durchlässige Schicht (300) ferner eine Hautkontakt-Verstärkungsschicht aufweist, die geeignet ist, benachbart zu einem Hautkontaktbereich an der Behandlungsstelle angeordnet zu werden, oder inhärente Hautkontaktverbesserungseigenschaften aufweist, die für das Stickoxid durchlässig ist, so dass bei ihrer Verwendung ein ausreichender Hautkontakt bereitstellt wird, und wobei die Hautkontaktverbesserungsschicht eine Klebstoffschicht ist, wie zum Beispiel eine Silikongelschicht, ein druckempfindlicher Klebstoff (pressure sensitive adhesive, PSA) oder eine Hydrogelschicht, die dazu geeignet ist, den Kontakt zwischen der stickoxidhaltigen Schicht (300) und dem Hautbereich bei der Verwendung zu verbessern.

4. Vorrichtung nach Anspruch 3, wobei die Hautkontaktverbesserungsschicht so konfiguriert ist, dass Unebenheiten in der Haut ausgefüllt werden, so dass ein Austreten von Stickoxid weg von der Stickoxid-Abgabevorrichtung statt in die Haut verhindert wird.

5. Vorrichtung nach Anspruch 3 oder 4, wobei die Hautkontaktverbesserungsschicht ausreichende Haftfähigkeiten bereitstellt, um die Stickoxid-Abgabevorrichtung an die Haut zu kleben.

6. Vorrichtung nach einem der Ansprüche 3 bis 5, wobei die Hautkontaktverbesserungsschicht an der NO-durchlässigen Schicht auf der Seite angebracht ist, die bei Gebrauch zur Haut hin gerichtet ist.

7. Vorrichtung nach einem der vorhergehenden Ansprüche, wobei die stickoxiddurchlässige Schicht (300) angepasst ist, um den Transport der unerwünschten Spezies von dem Behälter zu der Behandlungsstelle mittels ihrer Größe oder Löslichkeitsselektivität zu hemmen.

8. Vorrichtung nach einem der vorhergehenden Ansprüche, wobei das Reservoir (200) ein festes Medium umfasst, und wobei die Vorrichtung ferner eine im Wesentlichen Stickoxid-undurchlässige Trägerschicht umfasst, die angepasst ist, um einen erhöhten Partialdruck von gasförmigem Stickoxid, und folglich eine Konzentration des Stickoxids in dem Stickoxid freisetzenden Fluid, das in den festen Medien verteilt ist, die in dem Behälter in Kontakt mit der Stickoxid durchlässigen Schicht enthalten sind, die in Bezug auf Stickoxid im Wesentlichen in der Richtung des Behandlungsbereichs durchlässig ist, bereit zu stellen.

9. Vorrichtung nach Anspruch 8, wobei das Stickoxid freisetzende Fluid in dem festen Medium mit hoher Affinität für das Stickoxid freisetzende Fluid gleichmäßig verteilt ist, derart, dass ein hoher Grenzflächenbereich zwischen dem Stickoxid freisetzenden Fluid und der Stickoxid durchlässigen Schicht unter Umgebungsbeanspruchung der Vorrichtung, wie etwa Biegen, Anlegen eines ungleichen Drucks an die Vorrichtung und Kippen der Vorrichtung, bereitgestellt wird.

10. Topische Stickoxid-Abgabevorrichtung nach einem der vorhergehenden Ansprüche, umfassend
eine für Stickoxid im Wesentlichen undurchlässige Trägerschicht (100), die angeordnet und ausgelegt ist, um einen Verlust an Stickoxidkonzentration während der Freisetzung von Stickoxid aus dem Stickoxid freisetzenden Fluid in der Abgabevorrichtung zu begrenzen, wobei die stickoxiddurchlässige Schicht (300) bei der Verwendung der Abgabevorrichtung zu einem zu behandelnden Bereich ausgerichtet ist, und wobei das Reservoir eine absorbierende Schicht (200) ist, die zwischen der Trägerschicht (100) und der stickoxiddurchlässigen Schicht (300) angeordnet ist, wobei die absorbierende Schicht so angeordnet ist, dass Stickoxid freisetzendes Fluid darin in einer solchen Weise gehalten wird, dass Stickoxid, das von dem Stickoxid freisetzenden Fluid freigesetzt wird, innerhalb der Abgabevorrichtung und durch die stickoxiddurchlässige Schicht (300) durch eine Erhöhung des Partialdrucks oder der Konzentration von Stickoxid darin zu einem Hautbereich geleitet wird.

11. Topische Stickoxid-Abgabevorrichtung nach einem der vorhergehenden Ansprüche, wobei das Reservoir eine absorbierende Schicht (200) ist, die ein gelbasiertes Absorptionssystem eines Geliermaterials umfasst, das so konfiguriert ist, dass es ein Stickoxid freisetzendes Fluid absorbiert und festhält.

12. Vorrichtung nach einem der vorhergehenden Ansprüche, wobei das Reservoir ein Fluidreservoir ist, das in einer Absorptionsschicht (200) enthalten ist, das die Lagerung und den Einschluss des Stickoxid freisetzenden Fluids und eines Stickoxiddonor darin in einer Fluidstufe bereitstellt.

13. Vorrichtung nach einem der vorhergehenden Ansprüche, wobei die Stickoxid durchlässige Schicht (300) eine Membran ist, die selektiv für gasförmiges Stickoxid oder gelöstes Stickoxid durchlässig ist, wobei Stickoxid, das im Inneren der topischen Stickoxid-Abgabevorrichtung freigesetzt wird, auf die Behandlungsstelle gerichtet wird.

14. Topische Stickoxid-Abgabevorrichtung nach Anspruch 12, wobei das Fluidreservoir ein gelbildendes Material ist, das das Stickoxid freisetzende Fluid innerhalb seiner Grenzen enthält.

15. Vorrichtung nach Anspruch 11 bis 14, wobei die absorbierende Schicht (200) vollständig benetzt ist und wobei die absorbierende Schicht (200) einen Fluidgehalt von mehr als 60 %, wie zum Beispiel 80 % oder mehr als 90 %, aufweist.

16. Topische Stickoxid-Abgabevorrichtung nach einem der vorhergehenden Ansprüche, wobei die NO-permeable Schicht (300) konfiguriert ist, um die NO-Übertragungsgeschwindigkeit von der Vorrichtung zu der dermalen Behandlungsstelle zu regulieren, wobei die stickoxiddurchlässige Schicht (300) eine Membran aus Vlies oder porösem Material besteht, derart, dass die Stickoxid durchlässige Schicht (300) zum Transport eines Fluids konfiguriert ist, das NO darin gelöst aufweist und für eine gute Verbindung über die Stickoxid durchlässige Schicht (300) konfiguriert ist.

17. Vorrichtung nach einem der vorhergehenden Ansprüche,
wobei die stickoxiddurchlässige Schicht (300) aus einem Material mit hoher Feuchtigkeitsdampfübertragungsrate (MVTR), wie einer mikroporösen Membran oder einer hydrophilen Membran, die aus einer partiell hydrophilen Blockcopolymermembran besteht, hergestellt ist, so dass die stickoxiddurchlässige Schicht (300) zum Transport eines Fluids mit darin gelöstem NO und anderen nicht-toxischen, nicht reizenden Substanzen mit niedrigem Molekulargewicht über die Membran konfiguriert ist, aber größere Moleküle, z. B. das NO-Erzeugungssystem zurückhält,
konfiguriert für eine Medienkommunikation über die Stickoxid durchlässige Schicht (300).

18. Vorrichtung nach einem der vorhergehenden Ansprüche,
wobei die Stickoxid durchlässige Schicht (300) aus einem Medium oder einem Material mit niedrigem MVTR derart hergestellt ist, dass die Stickoxid durchlässige Schicht (300) zum Transport eines Fluids konfiguriert ist, das in sich NO gelöst aufweist und für eine niedrige Kommunikation über die stickoxiddurchlässige Schicht (300) konfiguriert ist.

19. Vorrichtung nach Anspruch 1 bis 17, wobei,
die stickoxiddurchlässige Schicht (300) so gewählt ist, dass sie für den NO-Transport hochselektiv ist, z. B. über einen Größen-Selektivitätsmechanismus arbeitet und keine Flüssigkeitskommunikation über die Membran zulässt.

20. Topische Stickoxid-Abgabevorrichtung nach einem der vorhergehenden Ansprüche, umfassend eine Klebstoffschicht (169) um einen Behandlungsbereich der topischen Stickoxid-Abgabevorrichtung, wobei die stickoxiddurchlässige Schicht innerhalb der Grenzen der Klebstoffschicht (169) angeordnet ist und wobei die Klebstoffschicht an der zu behandelnden Stelle klebriger ist als die Stickoxid durchlässige Schicht.

21. Topische Stickoxid-Abgabevorrichtung nach einem der vorhergehenden Ansprüche, umfassend ein Fluidrichtungssystem, das eine Stickoxiddonor-Verbindung enthält, die so angeordnet ist, dass sie das Aktivierungsfluid aufnimmt und das Aktivierungsfluid zu der Stickoxiddonor-Verbindung in der Weise lenkt, dass das verwendete Aktivierungsfluid die Stickoxiddonorverbindung vor dem Aufbringen auf die Absorptionsschicht (200) auflöst, wobei das Fluidrichtungssystem in dem Reservoir integriert ist.

22. Verwendung von Stickoxid bei der Herstellung einer Vorrichtung nach einem der Ansprüche 1 bis 21.

23. Verfahren zur kosmetischen Behandlung, umfassend die Abgabe von Stickoxid, wobei das Verfahren Folgendes umfasst:
Bereitstellen eines Stickoxid freisetzenden Fluids und Aktivieren der Stickoxidfreisetzung aus dem Stickoxid freisetzenden Fluid in einer Stickoxid-Abgabevorrichtung nach einem der Ansprüche 1 bis 21;
Platzieren der aktivierten Stickoxid-Abgabevorrichtung in Kontakt mit einem Hautbereich eines Körpers, wobei eine stickstoffdurchlässige Schicht (300) der Abgabevorrichtung zu dem Hautbereich ausgerichtet ist, wobei eine Permeabilität der stickoxiddurchlässigen Schicht (300) selektiv gewählt ist in Abhängigkeit von einem Toxizitätsniveau des Stickoxid-Fluids; und
topische Abgabe von Stickoxid von der Stickoxid-Abgabevorrichtung zu dem Hautbereich über die Stickoxid durchlässige Schicht (300) durch
eine Trägerschicht (100), die einen Verlust an Stickoxidkonzentration während der Freisetzung von Stickoxid aus dem Stickoxid freisetzenden Fluid begrenzt; und
eine absorbierende Schicht (200) zwischen der Trägerschicht (100) und der für Stickoxid durchlässigen Schicht (300), die das Stickoxid freisetzende Fluid darin zurückhält; wodurch ein Partialdruck oder eine Konzentration von Stickoxid in dem Stickoxid freisetzenden Fluid erhöht wird, und
Leiten von Stickoxid aus dem Stickoxid freisetzenden Fluid innerhalb der Abgabevorrichtung und durch die stickoxiddurchlässige Schicht (300) zu dem Hautbereich, und
Freisetzen des Stickoxids von dem Stickoxid-Donorsystem, derart, dass der Partialdruck und somit die Konzentration des gelösten Stickoxids in der Stickoxid-Abgabevorrichtung für die Abgabe von Stickoxid zunimmt, wobei der Hautbereich eine kosmetische Behandlungsstelle ist, wie zum Beispiel eine Narbenstelle, die durch die Hilfe von Stickoxid reduziert werden soll, oder eine Stelle von durchhängender Haut oder Falten, die durch das Stickoxid "geliftet" werden sollen.

24. Verfahren nach Anspruch 23, umfassend das Fixieren der Stickoxid-Abgabevorrichtung an dem Hautbereich mittels einer HautkontaktVerbesserungsschicht, die für Stickoxid durchlässig ist.

25. Verfahren zur Herstellung einer topischen Abgabevorrichtung nach einem der Ansprüche 1 bis 21, umfassend eine Filmumwandlung (170) und eine Montagefluidfüllung (172) in einer subatmosphärischen Druckumgebung oder einer Montagefluidfüllung (172) durch Füllen und Abdichten eines Flüssigkeitsbehälters und Anordnen des gefüllten Behälters in einer Umgebung unter Atmosphärendruck, wodurch ein sauerstofffreier und unter Atmosphärendruck unter Druck gesetzter Behälter geschaffen wird, der angeordnet ist, um das Stickoxid freisetzende Fluid zurückzuhalten.

26. Verfahren zur Herstellung einer topischen Abgabevorrichtung nach Anspruch 25, wobei die Filmumwandlung Folgendes umfasst:
Stanzen eines Hautklebstoffs, einer Membran und eines Absorptionsmittels von Folienrollen für einen unteren Teil der topischen Abgabevorrichtung,
Stanzen eines Fluidabteils von Folienrollen für einen oberen Teil der topischen Abgabevorrichtung,
Schweißen eines Fluidbeutels, und wobei die Montagefluidfüllung das Aufbringen einer Stickoxiddonorverbindung durch Sprühen oder Einspritzen in das Absorptionsmittel umfasst,
Aufbringen des Flüssigkeitsbeutels auf den oberen Teil der topischen Abgabevorrichtung,
Verschweißen des Flüssigkeitsbeutels mit dem unteren Teil der topischen Abgabevorrichtung in einer Unterdruckkammer, die das sauerstofffreie und unteratmosphärische unter Druck stehende enthaltene Absorptionsmittel erzeugt, und
Befüllen und Abdichten des Fluidbeutels.

## Revendications

1. Dispositif de distribution d'oxyde nitrique topique à disposer sur un site de traitement, comprenant
un fluide de libération d'oxyde nitrique utilisé dans ledit dispositif, dans lequel ledit fluide de libération d'oxyde nitrique comprend un fluide d'activation pour l'activation d'une libération d'oxyde nitrique à partir d'un composé donneur d'oxyde nitrique utilisé dans ledit dispositif, ledit composé donneur d'oxyde nitrique étant compris dans ledit dispositif de distribution d'oxyde nitrique ;
ledit composé donneur d'oxyde nitrique et ledit fluide d'activation sont séparés l'un de l'autre dans des réservoirs séparés par un film imperméable dans ledit dispositif durant le stockage dudit dispositif et conçus pour être mis en contact en retirant le film imperméable pour ladite libération d'oxyde nitrique à partir dudit fluide de libération d'oxyde nitrique, dans lequel ledit fluide de libération d'oxyde nitrique est au moins partiellement liquide ;
un réservoir (200) conçu pour contenir ledit fluide de libération d'oxyde nitrique ; et
une couche perméable à l'oxyde nitrique (300) adaptée pour venir au contact dudit site de traitement et adaptée pour fournir une zone d'interface pour le transport efficace dudit oxyde nitrique provenant dudit fluide de libération d'oxyde nitrique vers ledit site de traitement ; dans lequel au moins ladite couche perméable à l'oxyde nitrique est géométriquement flexible de telle sorte qu'une perte de ladite zone d'interface entre ladite couche perméable à l'oxyde nitrique et ladite zone de traitement est évitée lors de l'utilisation dudit dispositif,
ledit dispositif étant adapté pour fournir une distribution souhaitée, pouvant être régulée et constante dudit oxyde nitrique même sous l'effet d'une déformation physique dudit dispositif depuis ledit réservoir vers ledit site de traitement tout en évitant le transport d'espèces non souhaitées depuis ledit réservoir vers ledit site de traitement.

2. Dispositif selon la revendication 1, dans lequel le film imperméable est attaché à une bande (121) pour retirer le film imperméable entre les compartiments en tirant ladite bande (121).

3. Dispositif selon la revendication 1 ou 2, dans lequel ladite couche perméable à l'oxyde nitrique (300) comprend en outre une couche améliorant le contact avec la peau qui est disposée de manière adjacente à une zone de contact avec la peau au niveau dudit site de traitement, ou présente des propriétés inhérentes améliorant le contact avec la peau, qui est perméable audit oxyde nitrique, de sorte qu'il permette un contact avec la peau suffisant lors de son utilisation, et dans lequel ladite couche améliorant le contact avec la peau est une couche adhésive, comme une couche de gel de silicone, un adhésif sensible à la pression (PSA), ou une couche d'hydrogel, étant ainsi adaptée pour améliorer le contact entre ladite couche perméable à l'oxyde nitrique (300) et ladite zone de peau lors de son utilisation.

4. Dispositif selon la revendication 3, dans lequel ladite couche améliorant le contact avec la peau est configurée pour combler les irrégularités de la peau, de sorte que la fuite de l'oxyde nitrique hors dudit dispositif de distribution d'oxyde nitrique, plutôt qu'à l'intérieur de la peau, soit empêché.

5. Dispositif selon la revendication 3 ou 4, dans lequel ladite couche améliorant le contact avec la peau offre des capacités d'adhésion suffisantes pour coller ledit dispositif de distribution d'oxyde nitrique sur la peau.

6. Dispositif selon l'une quelconque des revendications 3 à 5, dans lequel ladite couche améliorant le contact avec la peau est attachée à la couche perméable à NO sur le côté qui est cours d'utilisation est tourné vers la peau.

7. Dispositif selon l'une quelconque des revendications précédentes, dans lequel ladite couche perméable à l'oxyde nitrique (300) est adaptée pour bloquer le transport desdites espèces non souhaitées depuis ledit réservoir vers ledit site de traitement grâce à la sélection de taille ou de solubilité de celle-ci.

8. Dispositif selon l'une quelconque des revendications précédentes, dans lequel ledit réservoir (200) comprend un support solide, et ledit dispositif comprenant en outre une couche de support sensiblement imperméable à l'oxyde nitrique adaptée pour fournir une pression partielle accrue d'oxyde nitrique gazeux et par conséquent une concentration dudit oxyde nitrique dans ledit fluide de libération d'oxyde nitrique distribué dans ledit support solide contenu dans ledit réservoir en contact avec ladite couche perméable à l'oxyde nitrique qui est perméable à l'oxyde nitrique sensiblement dans la direction de la zone de traitement.

9. Dispositif selon la revendication 8, dans lequel ledit fluide de libération d'oxyde nitrique est distribué de manière régulière dans ledit support solide à affinité élevée pour ledit fluide de libération d'oxyde nitrique, de sorte qu'une zone d'interface haute entre ledit fluide de libération d'oxyde nitrique et ladite couche perméable à l'oxyde nitrique soit prévue sous l'effet d'une contrainte environnementale dudit dispositif, par exemple le pliage, l'application d'une pression irrégulière sur le dispositif, et l'inclinaison du dispositif.

10. Dispositif de distribution d'oxyde nitrique topique selon l'une quelconque des revendications précédentes, comprenant
une couche de support (100) sensiblement imperméable à l'oxyde nitrique, disposée et configurée pour limiter une perte de concentration d'oxyde nitrique durant la libération d'oxyde nitrique provenant dudit fluide de libération d'oxyde nitrique dans le dispositif de distribution, dans lequel la couche perméable à l'oxyde nitrique (300) utilisée dans le dispositif de distribution est orientée vers une zone à traiter, et dans lequel ledit réservoir est une couche absorbante (200) disposée entre ladite couche de support (100) et ladite couche perméable à l'oxyde nitrique (300), la couche absorbante est conçue pour contenir ledit fluide de libération d'oxyde nitrique dans celle-ci de sorte que l'oxyde nitrique libéré dudit fluide de libération d'oxyde nitrique soit dirigé dans le dispositif de distribution et à travers la couche perméable à l'oxyde nitrique (300) vers une zone de peau par une augmentation de la pression partielle ou de la concentration d'oxyde nitrique dans celui-ci.

11. Dispositif de distribution d'oxyde nitrique topique selon l'une quelconque des revendications précédentes, dans lequel ledit réservoir est une couche absorbante (200) qui comprend un système d'absorption à base de gel d'une matière gélifiante, configuré pour absorber et contenir ledit fluide de libération d'oxyde nitrique.

12. Dispositif selon l'une quelconque des revendications précédentes, dans lequel ledit réservoir est un réservoir de fluide contenu dans une couche absorbante (200) qui permet le stockage et le piégeage dudit fluide de libération d'oxyde nitrique et d'un donneur d'oxyde nitrique dans celle-ci dans un étage de fluide.

13. Dispositif selon l'une quelconque des revendications précédentes, dans lequel ladite couche perméable à l'oxyde nitrique (300) est une membrane sélectivement perméable à l'oxyde nitrique gazeux ou à l'oxyde nitrique dissous, l'oxyde nitrique libéré à l'intérieur du dispositif de distribution d'oxyde nitrique topique étant dirigé vers ledit site de traitement.

14. Dispositif de distribution d'oxyde nitrique topique selon la revendication 12, dans lequel ledit réservoir de fluide est une matière gélifiante qui contient le fluide de libération d'oxyde nitrique dans ses limites.

15. Dispositif selon les revendications 11 à 14, dans lequel ladite couche absorbante (200) est entièrement humidifiée, et dans lequel ladite couche absorbante (200) comprend une teneur en fluide supérieure à 60 %, par exemple 80 % ou plus de 90 %.

16. Dispositif de distribution d'oxyde nitrique topique selon l'une quelconque des revendications précédentes, dans lequel ladite couche perméable à NO (300) est configurée pour réguler la vitesse de transfert de NO depuis le dispositif vers ledit site de traitement dermique, dans lequel ladite couche perméable à l'oxyde nitrique (300) est une membrane de matériaux non tissés ou poreux de sorte que ladite couche perméable à l'oxyde nitrique (300) soit configurée pour le transport d'un fluide ayant du NO dissous dans celle-ci et configurée pour une bonne communication à travers ladite couche perméable à l'oxyde nitrique (300).

17. Dispositif selon l'une quelconque des revendications précédentes,
dans lequel ladite couche perméable à l'oxyde nitrique (300) est constituée d'un matériau à taux de transfert de vapeur d'eau (MVTR) élevé, comme une membrane microporeuse ou une membrane hydrophile composée d'une membrane copolymère bloc partiellement hydrophile de sorte que ladite couche perméable à l'oxyde nitrique (300) soit configurée pour le transport d'un fluide ayant du NO dissous dans celle-ci et d'autres substances de faible poids moléculaire non irritantes et non toxiques sur la membrane mais contienne des molécules de plus grande taille, par exemple le système de génération de NO,
configuré pour une communication moyenne sur ladite couche perméable à l'oxyde nitrique (300).

18. Dispositif selon l'une quelconque des revendications précédentes,
dans lequel ladite couche perméable à l'oxyde nitrique (300) est constituée d'un matériau à MVTR moyen ou faible de sorte que ladite couche perméable à l'oxyde nitrique (300) soit configurée pour le transport d'un fluide ayant du NO dissous dans celle-ci et configurée pour une communication faible sur ladite couche perméable à l'oxyde nitrique (300).

19. Dispositif selon les revendications 1 à 17, dans lequel ladite couche perméable à l'oxyde nitrique (300) est choisie de sorte qu'elle soit hautement sélective vis-à-vis du transport de NO, par exemple fonctionnant par l'intermédiaire d'un mécanisme de sélection de taille, empêchant toute communication liquide sur la membrane.

20. Dispositif de distribution d'oxyde nitrique topique selon l'une quelconque des revendications précédentes comprenant une couche adhésive (169) autour d'une zone de traitement dudit dispositif de distribution d'oxyde nitrique topique, dans lequel ladite couche perméable à l'oxyde nitrique est disposée dans les limites de ladite couche adhésive (169) et dans lequel ladite couche adhésive est plus adhésive vers ledit site de traitement que ladite couche perméable à l'oxyde nitrique.

21. Dispositif de distribution d'oxyde nitrique topique selon l'une quelconque des revendications précédentes, comprenant un système de direction de fluide comprenant un composé donneur d'oxyde nitrique, conçu pour recevoir le fluide d'activation et diriger le fluide d'activation vers ledit composé donneur d'oxyde nitrique, de sorte que ledit fluide d'activation en cours d'utilisation dissolve ledit composé donneur d'oxyde nitrique avant de l'appliquer dans la couche absorbante (200), dans lequel ledit système de direction de fluide est intégré audit réservoir.

22. Utilisation d'oxyde nitrique dans la fabrication d'un dispositif selon l'une quelconque des revendications 1 à 21.

23. Procédé de traitement cosmétique comprenant la distribution d'oxyde nitrique, ledit procédé comprenant les étapes suivantes :
fournir un fluide de libération d'oxyde nitrique et activer une libération d'oxyde nitrique provenant dudit fluide de libération d'oxyde nitrique dans un dispositif de distribution d'oxyde nitrique selon l'une quelconque des revendications 1 à 21 ;
placer le dispositif de distribution d'oxyde nitrique activé en contact avec une zone de peau d'un corps, avec une couche perméable à l'oxyde nitrique (300) du dispositif de distribution orientée vers ladite zone de peau, dans lequel une perméabilité de ladite couche perméable à l'oxyde nitrique (300) est choisie sélectivement en fonction d'un niveau de toxicité dudit fluide d'oxyde nitrique ; et
distribuer l'oxyde nitrique par voie topique à partir dudit dispositif de distribution d'oxyde nitrique vers ladite zone de peau par l'intermédiaire de ladite couche perméable à l'oxyde nitrique (300) au moyen d'une couche de support (100) limitant une perte de concentration d'oxyde nitrique durant la libération de l'oxyde nitrique provenant dudit fluide de libération d'oxyde nitrique ; et
d'une couche absorbante (200) entre ladite couche de support (100) et
ladite couche perméable à l'oxyde nitrique (300) contenant ledit fluide de libération d'oxyde nitrique dans celle-ci ; augmentant ainsi une pression partielle ou une concentration d'oxyde nitrique dans ledit fluide de libération d'oxyde nitrique et
diriger l'oxyde nitrique provenant dudit fluide de libération d'oxyde nitrique à l'intérieur dudit dispositif de distribution et à travers ladite couche perméable à l'oxyde nitrique (300) vers ladite zone de peau, et
libérer ledit oxyde nitrique à partir dudit système donneur d'oxyde nitrique de sorte que ladite pression partielle, et donc une concentration d'oxyde nitrique dissous dans ledit dispositif de distribution d'oxyde nitrique,
augmente pour ladite distribution d'oxyde nitrique, dans lequel ladite zone de peau est un site de traitement cosmétique, comme un site de cicatrices devant être réduit par ledit oxyde nitrique, ou un site de peau affaissée ou
de rides à lisser au moyen dudit oxyde nitrique.

24. Procédé selon la revendication 23, consistant à fixer ledit dispositif de distribution d'oxyde nitrique à ladite zone de peau au moyen d'une couche améliorant le contact avec la peau perméable à l'oxyde nitrique.

25. Procédé de fabrication d'un dispositif de distribution topique selon l'une quelconque des revendications 1 à 21, comprenant la conversion de film (170), et le remplissage de fluide d'assemblage (172) dans un environnement sous pression sub-atmosphérique, ou le remplissage de fluide d'assemblage (172) en remplissant et en fermant hermétiquement un conteneur de fluide et en disposant le conteneur rempli dans un environnement sous pression sub-atmosphérique, créant ainsi un réservoir maintenu sous pression dépourvu d'oxygène et sub-atmosphérique conçu pour contenir ledit fluide de libération d'oxyde nitrique.

26. Procédé de fabrication d'un dispositif de distribution topique selon la revendication 25, dans lequel ladite conversion de film comprend les étapes suivantes
découper à l'emporte-pièce un ruban adhésif pour la peau, une membrane et une matière absorbante à partir de rouleaux de film pour une partie inférieure du dispositif de distribution topique,
découper à l'emporte-pièce un compartiment de fluide à partir de rouleaux de film pour une partie supérieure du dispositif de distribution topique, souder une poche de fluide, et dans lequel ledit le remplissage de fluide d'assemblage comprend les étapes suivantes
appliquer un composé donneur d'oxyde nitrique par pulvérisation ou injection sur ladite matière absorbante,
appliquer la poche de fluide sur la partie supérieure du dispositif de distribution topique,
souder la poche de fluide à la partie inférieure du dispositif de distribution topique dans une chambre de pression sous-atmosphérique créant ladite matière absorbante maintenue sous pression dépourvue d'oxygène et sub-atmosphérique, et
remplir et fermer hermétiquement ladite poche de fluide.
